# EUROPEAN PATENT SPECIFICATION

(11) **EP 1 649 061 B1**
(45) Date of publication and mention of the grant of the patent: **25.08.2010**
(21) Application number: 04777436.9
(22) Date of filing: 01.07.2004
(51) Int. Cl.: C12Q 1/68

(54) **GENES AS DIAGNOSTIC TOOLS FOR AUTISM**
GENE ALS DIAGNOSTISCHE WERKZEUGE FÜR AUTISMUS
GENES UTILES EN TANT QU'OUTILS DE DIAGNOSTIC POUR L'AUTISME

(30) Priority: 03.07.2003 US 484633 P
(43) Date of publication of application: 26.04.2006
(73) Proprietor: UNIVERSITY OF MEDICINE AND DENTISTRY OF NEW JERSEY, New Brunswick, NJ 08901 (US)
(72) Inventor: MILLONIG, James, H., Princeton Junction, NJ 08550 (US); BRZUSTOWICZ, Linda, Madison, NJ 07940 (US); GHARANI, Neda, Pennington, NJ 08534 (US)
(74) Representative: Peebles, Katrina
(86) International application number: PCT/US2004/021301
(87) International publication number: WO 2005/007812

(56) References cited:
- PETIT E ET AL: "ASSOCIATION STUDY WITH TWO MARKERS OF A HUMAN HOMEOGENE IN INFANTILE AUTISM" JOURNAL OF MEDICAL GENETICS, BMJ PUBLISHING GROUP, LONDON, GB, vol. 32, 1995, pages 269-274, XP008063984 ISSN: 0022-2593
- ZHONG H ET AL: "No association between the EN2 gene and autistic disorder" JOURNAL OF MEDICAL GENETICS, BMJ PUBLISHING GROUP, LONDON, GB, vol. 40, January 2003 (2003-01), pages 1-4, XP002992279 ISSN: 0022-2593
- LOGAN C ET AL: "Cloning and sequence comparison of the mouse, human, and chicken engrailed genes reveal potential functional domains and regulatory regions" DEVELOPMENTAL GENETICS, vol. 13, no. 5, 1992, pages 345-358, XP002435419 ISSN: 0192-253X
- LIU ET AL.: "A genomewide screen for autism susceptibility loci" AMERICAN JOURNAL OF HUMAN GENETICS, vol. 69, 2001, pages 327-340, XP002435420
- ALARCON ET AL.: "Evidence for a language quantitative trait locus on chromosome 7q in multiplex autism families" AMERICAN JOURNAL OF HUMAN GENETICS, vol. 70, 2002, pages 60-71, XP002435421
- PETIT E ET AL: 'Association Study With Two Markers of a Human Homeogene in Infantile Autism' J MED GENET vol. 32, 1995, pages 269 - 274, XP008063984
- ZEC N ET AL: 'Expression of the Homebox-containing Genes EN1 and EN2 in Human Fetal Midgestational Medulla and Cerebellum' JOURNAL OF NEUROPATHOLOGY AND EXPERIMENTAL NEUROLOGY vol. 56, no. 3, 1997, pages 236 - 242, XP008063988
- HONG S.S ET AL: 'DNA Polymorphisms of the Human Engrailed-like Genes, EN1 and EN2' MOL CELLS vol. 3, 1993, pages 101 - 107, XP008063998

## Description

### FIELD OF THE INVENTION

The present invention relates to autism spectrum disorder and methods for determining susceptibility to, or the presence of autism or a disease or discorder related thereto, such as Asperger's syndrome and Pervasive Developmental Delay-Not Otherwise Specified (PDD-NOS). More particularly, the invention relates to the identification of a susceptibility locus in autistic individuals, which may be used to diagnose developmental diseases, especially autism. Furthermore, the present invention comprises polymorphisms of the ENGRAILED 2 (EN2) gene, which correlate with a phenotype closely related to autism or a disease or disorder related thereto. The invention further relates to the identification of two single nucleotide polymorphisms (SNPs) that can be utilized for development of a genetic screening assay to assist in the diagnosis of autism. Such an assay can be used bo advise potential parents of their chances of having autistic children.

### BACKGROUND OF THE INVENTION

Autism spectrum disorder (AS) includes three separate diagnoses, which include autism, Asperger's syndrome and Pervasive Developmental Delay (PDD-NOS). PDD-NOS is characterized by developmental delays of sociability, communication and use imagination. Asperger's syndrome is a more severe form of PDD-NOS but lacks the language and intelligence deficits normally associated with autism. Autism is exemplified by severe communication impairments, social interaction deficits and repetitive/stereotypic behaviors. Each of these disorders has specific diagnostic criteria as outlined by the American Psychiatric Association (APA) in its *Diagnostic & Statistical Manual of Mental Disorders* (DSM-IV-TR).

Autism impacts the normal development of the brain in the areas of social interaction and communication skills. Children and adults with autism typically have difficulties in verbal and non-verbal communication, social interactions, and leisure or play activities.

There is no known single cause for autism, but it is generally accepted that it is caused by abnormalities in brain structure or function. Brain scans show differences in the shape and structure of the brain in autistic versus non-autistic children. Researchers are investigating a number of theories, including the link between heredity, genetics and medical problems. In many families, there appears to be a pattern of autism or related disabilities, further supporting a genetic basis to the disorder. While no one gene has been identified as causing autism, researchers are searching for genetic code variants that autistic children may have inherited. It also appears that some children are born with a susceptibility to autism.

Other researchers are investigating the possibility that under certain conditions, a number of genes with altered levels or functions may interfere with brain development resulting in autism. On the other hand, certain developmental genes may be associated with autism, including the Wnt2 and reelin genes have been implicated (Wassink, et al. Am. J. Med. Genet. 105:406-413 (2001); Persico, et al. Mol. Psychiatry 6:150-159 (2001)). Still other researchers are investigating problems during pregnancy or delivery as well as environmental factors such as viral infections, metabolic imbalances, and exposure to environmental chemicals.

PETIT, E. et al. (Association study with two markers of a human homeogene in infantile autism. Journal of medical genetics. 1995, vol. 32, pages 269-274) discloses a polymorphism in the 5' region of the EN-2 gene that is associated with autism.

ZHONG, H et al. (No association between the EN2 gene and autistic disorder. Journal of medical genetics. January 2003, vol. 40, pages 1-4) provides a study of the association between another EN-2 polymorphism and autism.

Autism tends to occur more frequently than expected among individuals who have certain medical conditions, including Fragile X syndrome, tuberous sclerosis, congenital rubella syndrome, and untreated phenylketonuria (PKU). Some harmful substances ingested during pregnancy also have been associated with an increased risk of autism. Early in 2002, The Agency for Toxic Substances and Disease Registry (ATSDR) prepared a literature review of hazardous chemical exposures and autism and found no compelling evidence for an association; however, there was very limited research and more needs to be done.

The question of a relationship between vaccines and autism continues to be debated. In a 2001 investigation by the Institute of Medicine, a committee concluded that the "evidence favors rejection of a causal relationship.... between MMR vaccines and autistic spectrum disorders (ASD)." The committee acknowledged, however, that "they could not rule out" the possibility that the MMR vaccine could contribute to ASD in a small number of children. While other researchers agree the data does not support a link between the MMR and autism, more research is clearly needed.

Whatever the cause, it is clear that children with autism and PDD-NOS are born with the disorder or born with the potential to develop it. Autism is not a mental illness. Children with autism are not unruly kids who choose not to behave. Furthermore, no known psychological factors in the development of the child have been shown to cause autism.

There are no medical tests for diagnosing autism. An accurate diagnosis must be based on observation of the individual's communication, behavior, and developmental levels. However, because many of the behaviors associated with autism are shared by other disorders, various medical tests may be ordered to rule out or identify other possible causes of the symptoms being exhibited.

A brief observation in a single setting cannot present a true picture of an individual's abilities and behaviors. Parental and other caregivers' input and developmental history are very important components of making an accurate diagnosis. At first glance, some persons with autism may appear to have mental retardation, a behavior disorder, problems with hearing, or even odd and eccentric behavior. To complicate matters further, these conditions can co-occur with autism. However, it is important to distinguish autism from other conditions, since an accurate diagnosis and early identification can provide the basis for building an appropriate and effective educational and treatment program.

Research indicates that early diagnosis is associated with dramatically better outcomes for individuals with autism. The earlier a child is diagnosed, the earlier the child can begin benefiting from one of the many specialized intervention approaches.

The present invention thus addresses the unmet need for the identification of an autism susceptibility locus and the uses for early diagnostic and prognostic purposes.

Accordingly, what is needed is a genetic marker to assess a subject's susceptibility to autism or a disease or disorder related thereto. Also needed is a genetic marker to diagnose autism, and the development of potential drugs or agents that have applications in treating autism or a disease or disorder related thereto, such as Asperger's Disorder or PDD-NOS.

### SUMMARY OF THE INVENTION

According to a first aspect of the present invention, there is provided an *in vitro* diagnostic method for determining the predisposition, the onset or the presence of autism spectrum disorder in a person, said method comprising detecting in said person the existence of a change in an intronic segment of the genome, which segment is located at least within the portion of chromosome 7 as set forth in SEQ ID NO:1, and wherein said change comprises a G to A transition at position 5579 and/or a T to C transition at position 5731 of SEQ ID NO: 1 .

According to a further aspect of the present invention, there is provided an in vitro method for determining whether a person has a genetic abnormality that predisposes such person to autism spectrum disorder, said method comprising either:
a) examining chromosome 7 of said person, and
   detecting, where present, the existence of a change in an intronic segment of the genome located on said chromosome 7,
   wherein said segment is located in SEQ ID NO:1,
   and wherein said change comprises a G to A transition at position 5579 and/or a T to C transition at position 5731 of SEQ ID NO: 1; or
b) detecting in tissue or cells from that person, the EN2 nucleic acid sequence as set forth in SEQ ID NO:1, comprising at least one single nucleotide polymorphism (SNP) in an intronic segment thereof wherein said SNP comprises a G to A transition at position 5579 and/or a T to C transition at position 5731 of SEQ ID NO:1.

An isolated nucleic acid molecule which encodes human ENGRAILED 2 (EN2), and the amino acid sequence of human EN2 is described. Furthermore, there is provided, in accordance with the present disclosure, methods for determining a subject's susceptibility to autism or a disease or disorder related thereto, such as Asperger's Disorder or PDD-NOS using a variant allele of the *ENGRAILED 2 (EN2)* gene, which maps to chromosome 7, in particular, to 7q36.3. Furthermore, transmission/disequilibrium tests (TDT), which were performed for two single nucleotide polymorphisms (*rs1861972* and *rs18619737*), revealed significant overtransmission of the A allele of *rs1861972* and the C allele of *rs1861973* (*rs1861972 P*=0.0009; *rs1861973 P*=0.0006). Haplotype analysis indicated that the A,C haplotype is specifically overtransmitted in antistic individuals (*P*=0.000062). Detection of such a variant allele in the genome of a subject may be indicative of the subject's susceptibility to autism. Furthermore, a variant allele of the *ENGRAILED 2* gene can also be used to assay drugs and agents for potential use in treating autism or a disease or disorder related thereto, such as Asperger's Disorder or PDD-NOS.

An isolated variant allele of a human ENGRAILED 2 gene (EN2) is described, wherein the ENGRAILED 2 gene comprises a DNA sequence of SBQ ID NO: 1, and the variant allele comprises a DNA sequence having at least one single nucleotide polymorphism, wherein the at least one variation comprises:
a G to A transition at position 154670848, hereinafter referred to as 154670848, the corresponding number when referring to the July 2003 assembly version # 69; or
a T to C transition at position 154671000, hereinafter referred to as 154671000, the corresponding number when referring to the July 2003 assembly version # 69; or
a combination thereof, and
wherein said variant allele correlates with the predisposition, the onset or the presence of autism spectrum disorder in a mammal.

A diagnostic method for determining the predisposition, the onset or the presence of autism spectrum disorder in a mammal is described, said method comprising detecting in said mammal the existence of a change in a segment of the genome, that segment located within the portion of chromosome 7 as set forth in SEQ ID NO: 1. The segment may comprise the human EN2 gene and is located on chromosome 7 from about position 154552000 to about position 154560000. The autism spectrum disorder may be selected from the group consisting of autism, Asperger's disorder and Pervasive Development Delay (PDD-NOS). The segment of the genome may contain a variant allele or mutation that predisposes individuals to Autism Spectrum Disorder, and this variant allele or mutation may be a deletion, an addition and a substitution of at least one nucleotide. Furthermore, the substitution may be a single nucleotide polymorphism (SNP) located at position 154670848. This substitution may comprise a G to A transition at position 154670848. Furthermore, the substitution may be a single nucleotide polymorphism (SNP) located at position 154671000. This substitution may comprise a T to C transition at position 154671000, or the substitution may be a combination of both SNPs.

A detectably labeled isolated variant allele of a human EN2 gene is described, wherein the EN2 gene comprises a DNA sequence of SEQ ID NO: 1, and the variant allele comprises a DNA sequence having at least one variation in SEQ ID NO:1, wherein the at least one variation comprises:
a G to A transition at position 154670848; or
a T to C transition at position 154671000 or
a combination thereof.

Numerous detectable labels have applications. For example the detectable label can be a radioactive element, such as the isotopes ³H, ¹⁴C, ³²P, ³⁵S, ³⁶Cl, ⁵¹Cr, ⁵⁷Co, ⁵⁸Co, ⁵⁹Fe, ⁹⁰Y, ¹²⁵I, ^{131I}, and ¹⁸⁶Re, to name only a few. Chemicals which fluoresce, or enzymes such as alkaline phosphatase or horseradish peroxidase, can also be used as detectable labels.

A diagnostic method for determining that a person has a genetic abnormality that predisposes that person, or his or her offspring, to autism spectrum disorder, including autism, Asperger's disorder, or PDD-NOS is described, wherein said genetic abnormality is found on chromosome 7, said method comprising detecting, in that person, the existence of a change in a segment of the genome, that segment located within the portion of chromosome 7 as set forth in SEQ ID NO: 1. The segment comprises the human EN2 gene which is located on chromosome 7 from about position 154552000 to about position 154560000, said segment having the sequence set forth is SEQ ID NO: 1. The person diagnosed may not be autistic (i.e. may not exhibit any autistic symptoms or behaviors), however, if the person diagnosed has the genetic abnormality that predisposes that person to autism, then this diagnosis signifies that the person diagnosed could bear children who are predisposed to autism. The determination can be made before the person is born, as early as the blastocyst stage of embryonic development and throughout the fetal stage, or at any time after the person is born. It is sufficient that the abnormality occur in one chromosome of a person; i.e., the person can be heterozygous as regards the abnormality. Also described is a method for determining whether a person has a genetic abnormality that predisposes such person to autism spectrum disorder, said method comprising:
examining chromosome 7 of said person, and
detecting, where present, the existence of a change in a segment of the genome located in said chromosome 7,
wherein said segment is set forth in SEQ ID NO:1.

The diagnostic method involves detecting changes in the ENGRAILED 2 (*EN2)* gene. Changes (or variant alleles) are additions, deletions, or substitutions of at least one nucleotide. Thus, the diagnostic method comprises detecting a change in a region of chromosome 7 spanning the *EN2* gene. In the context of this application, the *EN2* gene means the sequence necessary for transcription and translation such that normal levels of functional *EN2* protein are generated. The substitution may be a single nucleotide polymorphism (SNP) located at position 154670848, and the substitution may consist of a guanine (G) to adenine (A) transition. Alternatively, the substitution may be a single nucleotide polymorphism (SNP) located at position 154671000 and may consist of a thymine (T) to cytosine (C) transition. In addition, since cis-regulatory regions needed for *EN2* expression could be, for example, as far as 1000 kb away from the coding sequence of the gene, the *EN2* gene also means a region on human Chromosome 7 of approximately 154010087-155102101 base pairs and encompasses the *EN2* coding region (approximately 8000 base pairs) and flanking sequence.

A diagnostic method useful for determining that a person has a genetic abnormality that predisposes that person to autism spectrum disorder is described, said method comprising detecting in that person the existence of a mutation in the *EN2* gene such that said mutation results in the deletion, addition, or substitution of at least one amino acid in the EN2 protein.

Another example of the EN2 gene are the single nucleotide polymorphisms (SNPs) comprising a region amplifiable by polymerase chain reaction, wherein said region is amplified using a primer pair, wherein a primer is selected from the group consisting of the sequences set forth is SEQ ID NOs: 3, 4, 5, 6, 7, 8, 9 and 10. Accordingly, a diagnostic method for determining that a person has a genetic abnormality that predisposes that person to autism is described, said method comprising detecting, in that person, the existence of a change, ie. a deletion, an addition or a substitution in a segment of the genome, that sequence located within the portion of chromosome 7 set forth in SEQ ID NO: 1 (the EN2 gene), and amplifying portions of that sequence containing the SNPs identified herein, using primer pairs selected from the group consisting of the sequences set forth is SEQ ID NOs: 3, 4, 5, 6, 7, 8, 9 and 10.

Primer sequences that flank the EN2 transcript (i.e. the coding region) on chromosome 7 are:

rs1861972
Forward outer 5' TGAAGCTGGGGCCAGATGCTCCTA3' (SEQ ID NO:3) and
Reverse outer- 5'CATGGGGAAAGGGCAGAGGGAGGAG3' (SEQ ID NO:4)
Forward inner- 5'AGGCGAGGTCACCACTCCCTGCAAG3' (SEQ ID NO: 5) and
Reverse inner- 5'GGGGGAAGAAGGGGGCAAGGCAAT3' (SEQ ID NO: 6)

rs1861973
Forward outer 5' GCCAGGGGGTTGAGCCTCTTAT3' (SEQ ID NO: 7)
Reverse outer 5'CAGGTCCACTTCTGACCCTCC3' (SEQ ID NO: 8)
Forward inner 5'GAAGCCTTACAGCGACCCGGT3' (SEQ ID NO: 9) and
Reverse inner 5'GACCTGCCCCAGGTTTTGG3'(SEQ ID NO: 10)

Within that region is the coding region fo the *EN2* gene. One or more single nucleotide polymorphisms (SNPs) in the *EN2* gene are a preferred indicator of a predisposition to autism. Such SNPs may occur anywhere in the *EN2* gene as defined above. Alternatively, a deletion in the coding region would affect the sequence of the expressed protein. A deletion in the 5' or 3' noncoding region would affect expression levels or RNA stability. A deletion 5' or 3' of the exons that encode EN2 mRNA would affect expression levels by perturbing the promoter or enhancers. Any of these deletions may have an impact on the expression product of the *EN2* gene and consequently cause predisposition to autism.

Any deleted segment of one or more bases in size is diagnostically significant. Accordingly, a deletion of a single nucleotide is diagnostically significant, in particular this or any deletion that changes the function, activity, or expression levels of the EN2 protein. The deletion can be as large as a deletion of the entire *en2* gene, or any portion of it.

Other changes (meaning in general mutations, but including any possible difference in the sequence of the gene from the wild-type) in the en2 gene as defined above is described. In addition to deletions as discussed above, these changes may involve additions or substitutions (by which is meant replacement of a given nucleotide or amino acid by another nucleotide or amino acid). The size or extent of the addition or substitution may be one nucleotide, to any size or extent which changes or disrupts EN2 expression as described above. Any one, two, or three of these changes, or mutations, may occur in one individual. Particular changes, or mutations, of interest are those that alter the function, activity, or expression levels of the EN2 protein. These additions or substitutions may be in the coding region, and thus would affect the expressed EN2 protein as described immediately below. The additions or substitutions may also be in the 5' or 3' noncoding region, in the introns, and accordingly would affect protein expression equivalently to the effect of deletions in these regions as described immediately above. Additions or substitutions in the areas spanned by the sequence pairs provided above are particularly of interest. Accordingly, a diagnostic method for determining that a person has a genetic abnormality that predisposes that person to autism is described said method comprising detecting, in that person, the existence of an addition or deletion of at least one nucleotide to a segment of the genome, that segment located within the portion of chromosome 7 as set forth is SEQ ID NO: 1, or comprising detecting, in that person, the existence of a substitution of at least one nucleotide for another nucleotide in a segment of the genome, that segment located within the portion of chromosome 7 as set forth in SEQ ID NO: 1. Such changes (i.e. deletions, additions, or substitutions) affect EN2 function, since they occur in some segment of the *en2* gene.

Further, of particular interest are diagnostic methods where the changes (deletions, additions, or substitutions) are detected within the portion of chromosome 7, in an intron, or in a segment of an *en2* gene, defined by the sequences selected from the group consisting of SEQ ID NOs 3, 4, 5, 6, 7, 8, 9, and 10.

Detecting a mutation in an exon, in any chromosome region or segment specified above is described, providing the mutation results in a change in an amino acid in the protein coded for. Thus, detecting the existence of a mutation in the en2 gene such that the mutation results in the deletion, addition, or substitution of at least one amino acid in the EN2 protein is described.

Detecting deletions or mutations in the proteins coded for by the segments specified above is described, especially the EN2 protein (SEQ ID NO: 2). Thus detecting a change with respect to at least one amino acid in, for example, the EN2 protein, which change can be one or more additions, deletions, or substitutions, or a combination thereof (the protein in question maintains its identity by virtue of being encoded by the locus which encodes that protein, for example oven if there are multiple changes in that EN2 protein, it is still considered the EN2 protein in the context of this invention as it is encoded by the *en2* gene) is described.

A diagnostic method for determining a subject's susceptibility to autism or a related disorder, comprising measuring the presence or absence of the A allele of *rs1861972* and/or the presence or absence of the C allele of *rs1861973* is described.

A diagnostic method comprising detecting, in tissue or cells from a person, an increase or a decrease in the level of normal RNA (mRNA and/or hnRNA) transcribed from a region of SEQ ID NO: 1, for example those segments specified by the sequences set forth above is described. By normal RNA is meant RNA which encodes a protein which does not have an addition, deletion, or substitution, a wild-type protein, in particular which encodes such a protein which is an EN2 protein. Thus, the diagnostic method may comprise detecting abnormal RNA, which means RNA that is longer than normal RNA (i.e. has at least one addition), shorter than normal RNA (i.e. has at least one deletion), or is different than normal RNA (i.e. has at least one substitution, or has an equal number of deletions and additions).

One example is methods for diagnosing autism and/or Asperger's disorder and/or PDD-NOS and clinically related conditions. This includes the use of the genes or gene products identified by the methods described herein, including but not limited to those genes and gene products identified through use of a mammalian model of autism. In a preferred example , the gene useful for diagnosis of autism, Asperger's or PDD-NOS and other clinically related conditions includes the gene identified in the nucleic acid of SEQ ID NO: 1 (EN2).

Another example includes the use of the gene products, in particular, the protein identified by the methods described herein, including but not limited to the gene product identified as the protein of SEQ ID NO: 2.

A method of determining if a subject is at risk for developing autism and/or Asperger's and/or PDD-NOS is described, said method comprising:
(I) measuring an amount of an EN2 gene or gene product in a tissue sample derived from the subject, wherein said gene or gene product is:
   (a) a DNA corresponding to SEQ ID NO: 1, or a nucleic acid derived therefrom;
   (b) a protein comprising SEQ ID NO: 2;
   (c) a nucleic acid comprising a sequence hybridizable to SEQ ID NO: 1, or its complement under conditions of high stringency, or a protein comprising a sequence encoded by aid hybridizable sequence;
   (d) a nucleic acid at least 90% homologous to SEQ ID NO: 1, or its complement as determined using the NBLAST algorithm; or a protein encoded thereby; and
(II) comparing the amount of said gene product in the subject with the amount of gene product present in a normal tissue sample (taken from a non-autistic individual) or predetermined standard for a normal tissue sample, wherein an elevated amount of said gene product in the subject compared to the amount in the normal tissue sample or predetermined standard for a normal tissue sample indicates a risk of developing autism and/or Asperger's and/or PDD-NOS in the subject.

A further example is a method for screening, diagnosis or prognosis of AS selected from the group consisting of autism, Asperger's and PDD-NOS, said method comprising:
(I) measuring an amount of an EN2 gene or gene product in a tissue sample derived from the subject, wherein said gene or gene product is:
   (a) a DNA corresponding to SEQ ID NO: 1, or a nucleic acid derived therefrom;
   (b) a protein comprising SEQ ID NO: 2;
   (c) a nucleic acid comprising a sequence hybridizable to SEQ ID NO: 1, or its complement under conditions of high stringency, or a protein comprising a sequence encoded by said hybridizable sequence;
   (d) a nucleic acid at least 90% homologous to SEQ ID NO: 1, or its complement as determined using the NBLAST algorithm; or a protein encoded thereby; and
(II) comparing the amount of said gene product in the subject with the amount of EN2 gene product present in a non-autistic tissue sample or predetermined standard for a non-autistic tissue sample, wherein an elevated amount of said EN2 gene product in the subject compared to the amount in the non-autistic tissue sample or pre-determined standard for a non-autistic tissue sample indicates a risk of developing autism and/or Asperger's and/or PDD-NOS in the subject.

In another example , the diagnostic method comprises detecting, in tissue or cells from a person, an increase or a decrease in the level of normal EN2 protein, for example, protein coded for a by region of chromosome 7 set forth as SEQ ID NO: 1, in particular a segment, or region specified above, for example those segments specified by the sequences set forth above. By normal protein is meant a protein which does not have an addition, deletion, or substitution, a wild-type protein, in particular an EN2 protein. Thus the diagnostic method may comprise detecting abnormal protein, which means a protein that is longer than the normal protein (i.e. has at least one addition), shorter than the normal protein (i.e. has at least one deletion), or different than the normal protein (i.e. has at least one substitution, or has an equal number of deletions and additions).

Overexpression of EN2 protein may also indicate autism or predisposition to autism. Accordingly, the diagnostic method may comprise detecting, in tissues or cells from a person, an increased level of normal RNA transcribed from a region of chromosome 7 set forth is SEQ ID NO: 1, in particular a segment, or region, for example those segments specified by the sequences set forth above.

In a related protein-based assay, the diagnostic method comprises detecting, in tissue or cells from a person, an increased level of normal EN2 protein, for example, protein coded for a by a region of chromosome 7 set forth in SEQ ID NO: 1, in particular a segment, or region, for example, those segments specified by the sequences set forth above.

By increased or decreased levels of RNA or protein is meant levels that differ from levels found in a normal person with no predisposition to autism. Accordingly, the normal level of RNA or protein may be determined by measuring RNA or protein levels from a sufficiently large sample of the normal population (about N = 100 or greater). Since there will be a certain amount of variability, conventional statistical analysis may be used to establish a mean for the normal level of RNA or protein. An increased level of RNA or protein is a level which is significantly higher than the mean normal level by statistical analysis. A decreased level of RNA or protein is a level which is significantly lower than the mean normal level by statistical analysis. For example, a level of 10% higher or lower than normal may be considered an increased or decreased level of RNA or protein.

A diagnostic kit is described, comprising a nucleic acid hybridization probe specific for part or all of a region of the gene encoding en2, or a segment thereof. Alternatively, the kit comprises PCR primers that can be used to amplify part of a chromosome that falls within a region so specified (e.g. chromosome 7). In particular, such kits contain the PCR primers specified above, suitably paired. The kit may be a protein-based kit comprising an antibody probe specific for all or part of the EN2 protein.

An antibody is described that recognizes and binds to the amino acid sequence of SEQ ID NO: 2, encoded by the normal EN2 nucleic acid sequence and/or the nucleic acid sequence of SEQ ID NO: 1, which contains the A allele of rs1861972 located at position 154670848 and/or the C allele of rs1861973 located at position 154671000 of SEQ ID NO: 1, conservative variants thereof, fragments thereof, or analogs or derivatives thereof, as an immunogen. Such an antibody can be polyclonal, monoclonal, single chain, or chimeric. They may be human, mouse, rat, rabbit, goat, horse, sheep or may be obtained from other non-human animals. Further, an antibody having human EN2 as an immunogen can be detectably labeled. As explained above, examples of detectable labels include, but certainly are not limited to radioactive isotopes, such as ³H, ¹⁴C, ³²P, ³⁵S, ³⁶Cl, ⁵¹Cr, ⁵⁷Co, ⁵⁸Co, ⁵⁹Fe, ⁹⁰Y, ¹²⁵I, ¹³¹I, and ¹⁸⁶Re, to name only a few. Chemicals which fluoresce, or enzymes such as alkaline phosphatase or horseradish peroxidase, can also be used as detectable labels. These antibodies may be utilized for diagnostic or therapeutic purposes.

A method of treating a subject with autism or an autism related disorder such as Asperger's or PDD-NOS, or a person predisposed to a developmental disorder (especially autism) is described, the method comprising either administering a gene or a protein to the person. The method of treating a subject may comprise the step of introducing a DNA molecule coding for a polypeptide normally coded for (e.g., in non-autistic patients) by a gene located within the chromosome 7 region set forth in SEQ ID NO: 1. The polypeptide may be the EN2 protein, and the disorder may be autism.

The DNA molecule may be introduced into a cell and the cell administered to the person (postnatal or prenatal). The cell, may for example, be a stem cell. The stem cell into which a DNA molecule is introduced for therapeutic purposes is preferably a stem cell isolated from the person who will receive the stem cell containing the DNA molecule encoding the EN2 protein. The stem cell can, for example, be obtained from that person's bone marrow. Alternatively, the stem cell can be obtained from another person. An additional alternative is that the stem cell be obtained from an animal, especially a mammal.

The therapeutic process may comprise the step of introducing the EN2 protein into a person.

The therapeutic process may also comprise the step of administering a drug to a person so as to normalize (i.e. increase or decrease to normal levels) the level of EN2 protein in a tissue in said person.

The therapeutic process may also comprise the step of administering a drug so as to normalize (i.e. increase or decrease to normal levels) the level of genes that are activated by EN2 protein.

Any of these therapeutic processes may be used in particular to treat autism or a predisposition to autism or an autism related disorder such as Asperger's or PDD-NOS.

Pharmaceutical compositions comprising a therapeutically effective amount of the genes or gene products are described, including but not limited to the nucleic acid of SEQ ID NO: 1 or the protein of SEQ ID NO: 2, with a pharmaceutically acceptable carrier for delivery to an individual in need of such therapy. Included are agonists or antagonists of the gene or gene products identified herein. Such agonists or antagonists may be small synthetic organic molecules, proteins, peptides, polypeptides or antibodies. Further, a therapeutically effective amount of an agent that modulates the expression aud/or activity of the EN2 gene or gene product and a pharmaceutically acceptable carrier is described. The pharmaceutical compositions may be delivered orally, intravenously, intramuscularly, subcutaneously. intrathecally, intracranially. They may be in the form of tablets, capsules, suspensions, suppositories or in liquid form suitable for intravenous delivery.

### BRIEF DESCRIPTION OF THE FIGURES

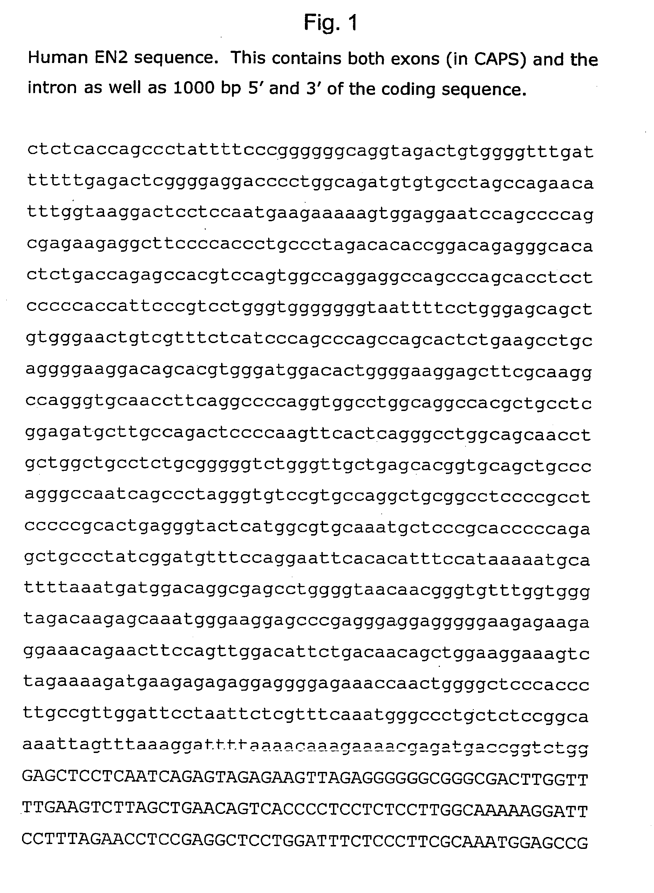

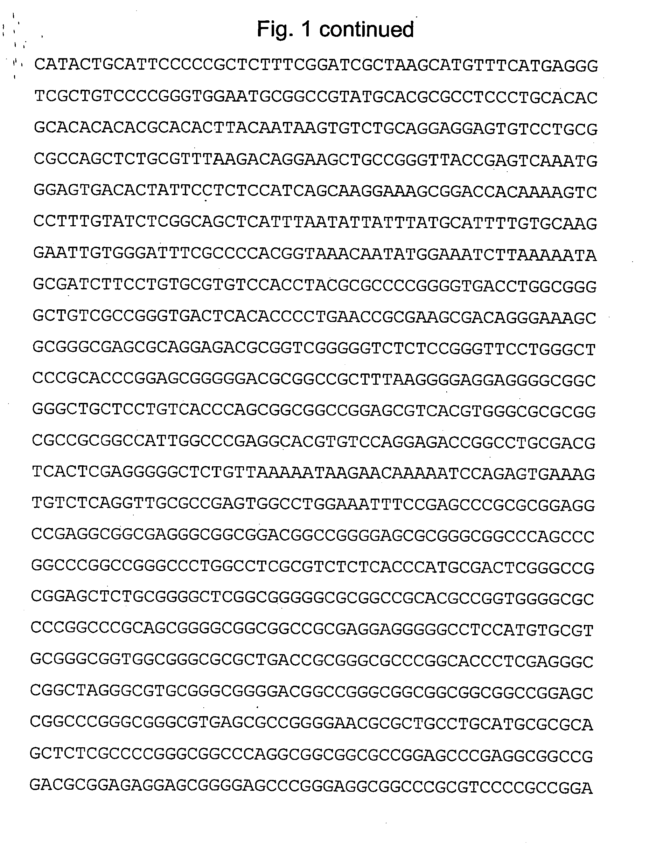

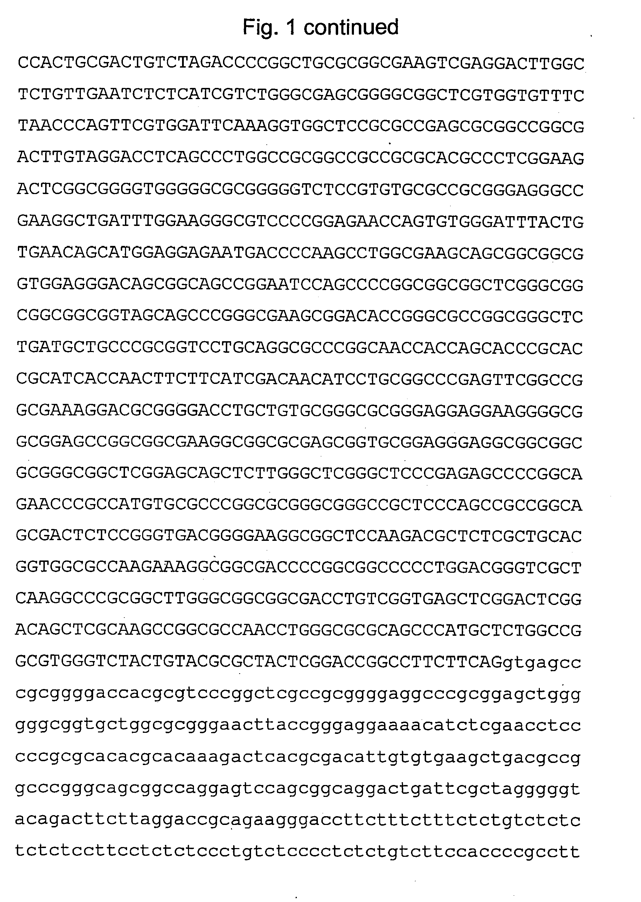

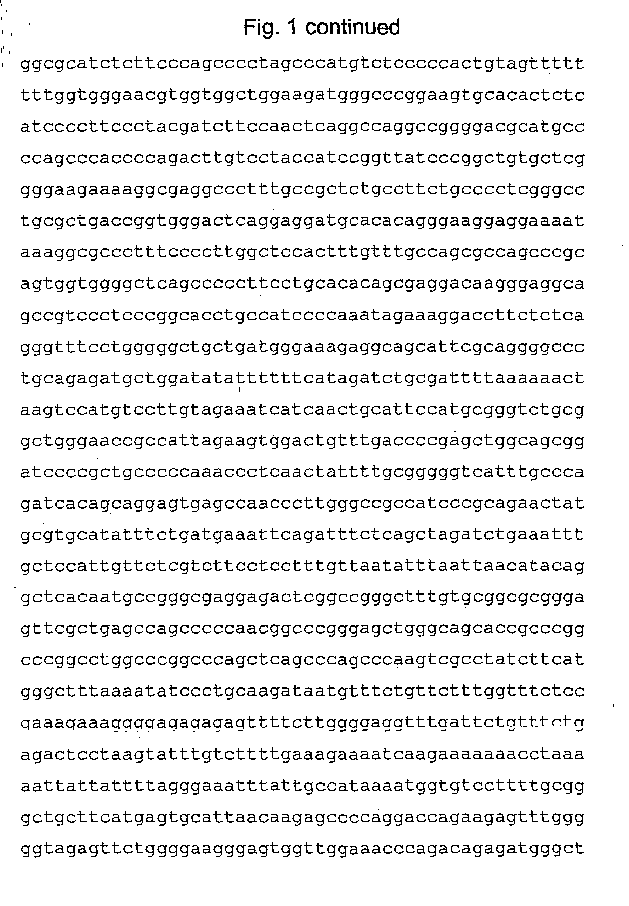

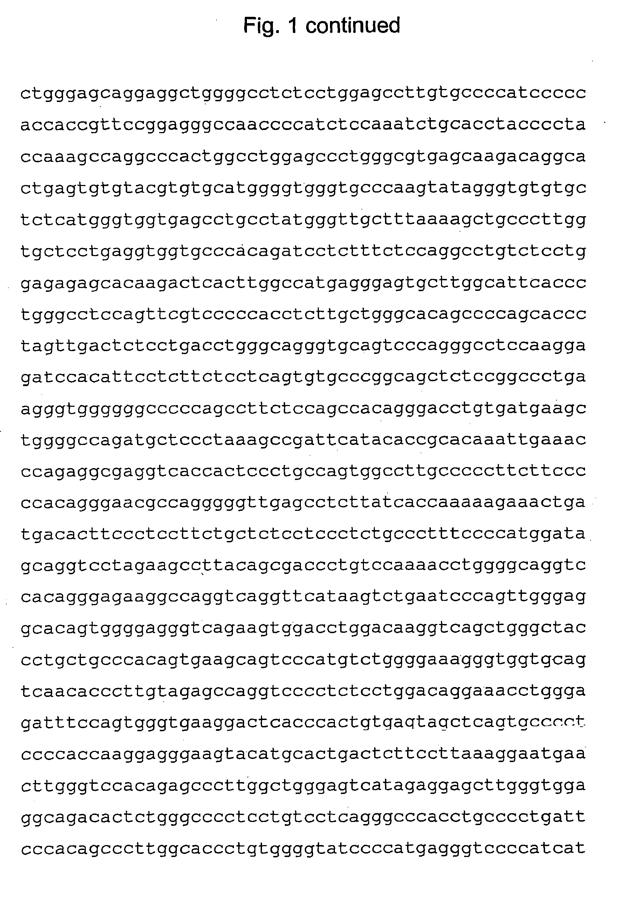

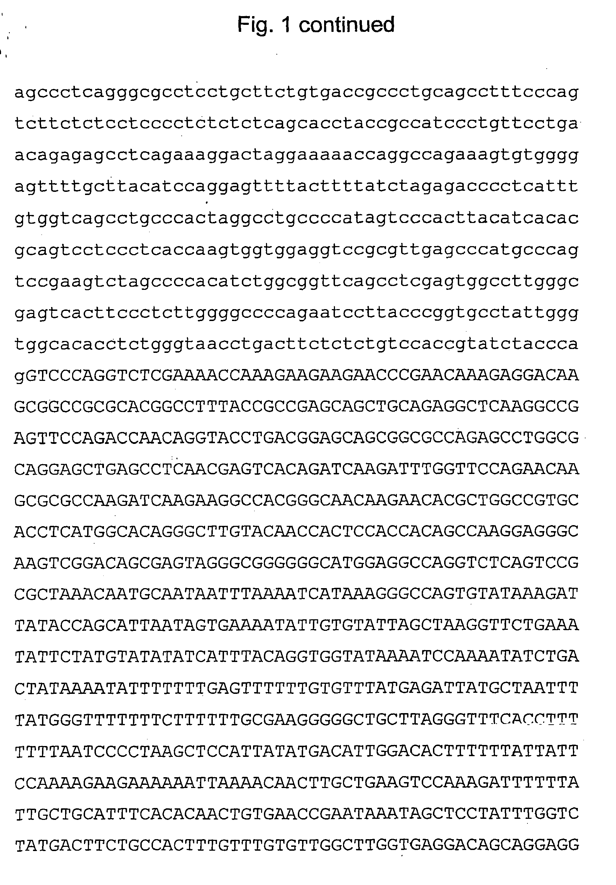

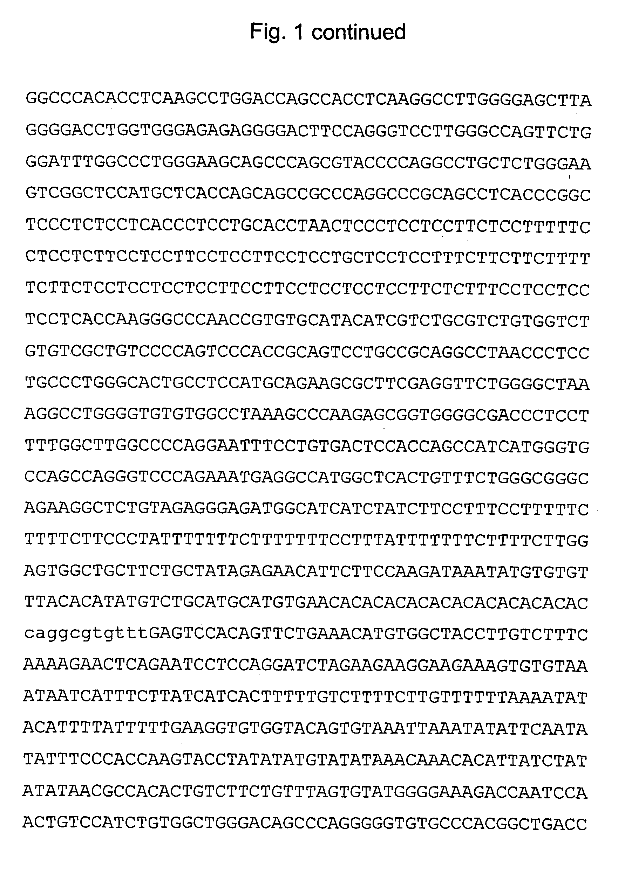

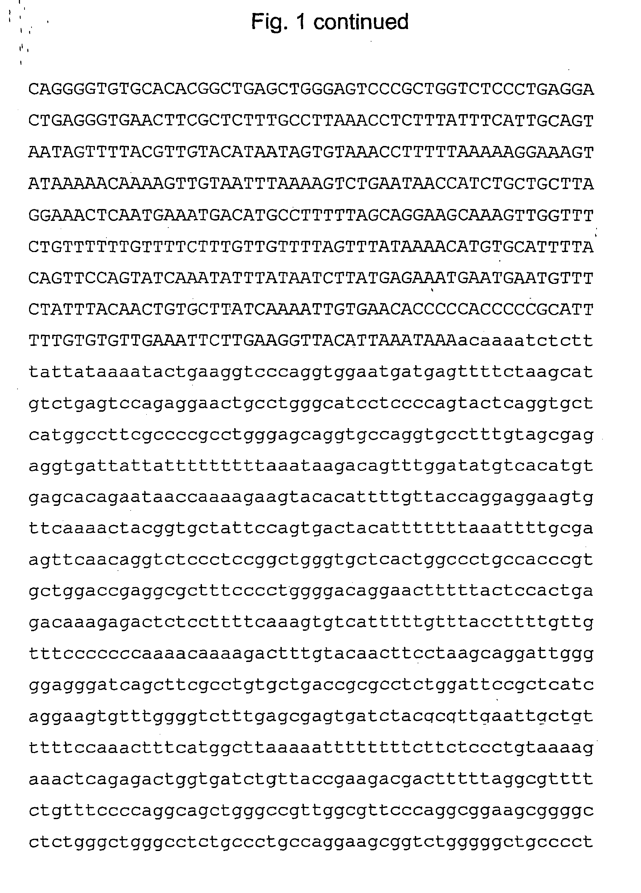

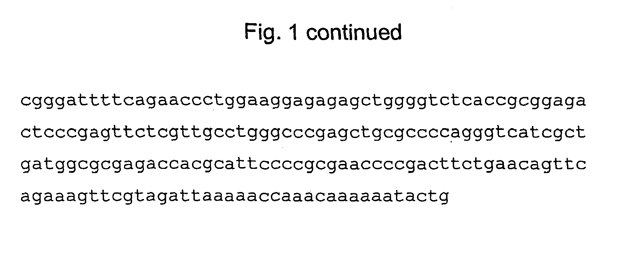

**Figure 1**. DNA Sequence Encoding Human EN2

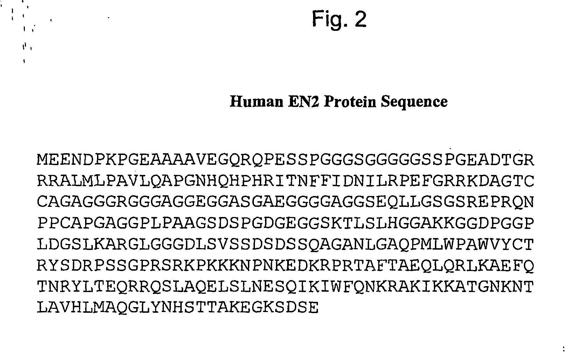

**Figure 2**. Protein Sequence for Human EN2

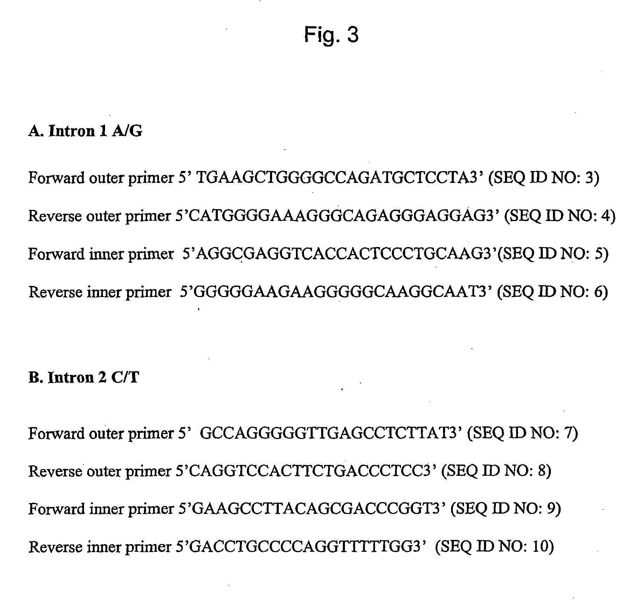

**Figure 3**. Forward and Reverse Primers for rs1861972 and rs1861973

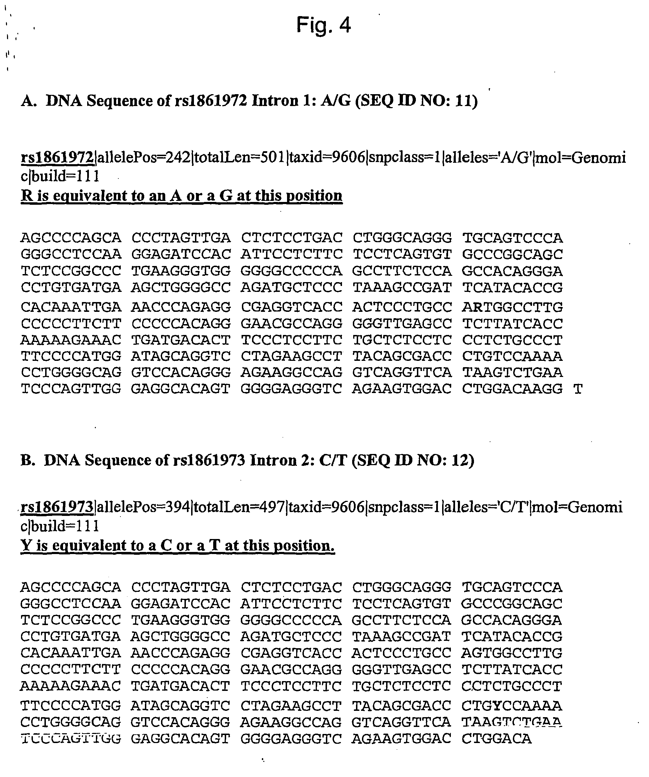

**Figure 4**. DNA Sequences for SNP NOs. rs1861972 and rs1861973

**Figure 5**. Schematic Representation of the Human *ENGRAILED 2* gene

**Figure 6**. Schematic illustration of inter-marker linkage disequilibrium values

### DETAILED DESCRIPTION

Before the present methods and treatment methodology the described, it is to be understood that this invention is not limited to particular methods, and experimental conditions described, as such methods and conditions may vary. It is also to be understood that the terminology used herein is for purposes of describing particular embodiments only, and is not intended to be limiting, since the scope of the present invention will be limited only in the appended claims.

As used in this specification and the appended claims, the singular forms "a", "an", and "the" include plural references unless the context clearly dictates otherwise. Thus, for example, references to "the method" includes one or more methods, and/or steps of the type described herein and/or which will became apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

### Definitions

The terms used herein have the meanings recognized and known to those of skill in the art, however, for convenience and completeness, particular terms and their meanings are set forth below.

"Treatment" refers to the administration of medicine or the performance of medical procedures with respect to a patient, for either prophylaxis (prevention) or to cure the infirmity or malady in the instance where the patient is afflicted.

A "therapeutically effective amount" is an amount sufficient to decrease or prevent the symptoms associated with autism associated with the presence of at least one of the SNPs identified in the presence invention.

The term "antibody" as used herein includes intact molecules as well as fragments thereof, such as Fab and F(ab')₂, which are capable of binding the epitopic determinant. Antibodies that bind EN2 can be prepared using intact polypeptides or fragments containing small peptides of interest as the immunizing antigen attached to a carrier molecule. Commonly used carriers that are chemically coupled to peptides include bovine serum albumin and thyroglobulin. The coupled peptide is then used to immunize the animal (e.g, a mouse, rat or rabbit).

"Gene Product" as used herein, unless otherwise indicated, is a protein or polypeptide encoded by the nucleic acid sequences identified by the methods described herein, including but not limited to SEQ ID NO: 1; a nucleic acid comprising a sequence hybridizable to SEQ ID NO: 1 or its complement under conditions of high stringency, or a protein comprising a sequence encoded by said hybridizable sequence; a nucleic acid at least 90% homologous to SEQ ID NO:1 or its complement as determined using the NBLAST algorithm; a nucleic acid at least 90% homologous to SEQ ID NO:1 or a fragment or derivative of any of the foregoing proteins or nucleic acids.

A "variant" (v) of polynucleotides or polypeptides, as the term is used herein, are polynucleotides or polypeptides that are different from a reference polynucleotide or polypeptide, respectively. Variant polynucleotides are generally limited so that the nucleotide sequence of the reference and the variant are closely related overall and, in many regions, identical. Changes in the nucleotide sequence of the variant may be silent. That is, they may not alter the amino acid sequence encoded by the polynucleotide. Where alterations are limited to silent changes of this type a variant will encode a polypeptide with the same amino acid sequence as the reference. However, silent variants can affect protein levels by codon use. Thus, in this particular situation, different tRNAs may vary in abundance so that even though the change is silent, it may still be functionally important. Alternatively, changes in the nucleotide sequence of the variant may alter the amino acid sequence of a polypeptide encoded by the reference polynucleotide. Such nucleotide changes may result in amino acid substitutions, additions, deletions, fusions, and truncations in the polypeptide encoded by the reference sequence. Variant polypeptides are generally limited so that the sequences of the reference and the variant are that are closely similar overall and, in many regions, identical. For example, a variant and reference polypeptide may differ in amino acid sequence by one or more substitutions, additions, deletions, fusions, and truncations, which may be present or absent in any combination. Such variants can differ in their amino acid composition (*e.g.* as a result of allelic or natural variation in the amino acid sequence, *e*.*g*. as a result of alternative mRNA or pre-mRNA processing, *e*.*g*. alternative splicing or limited proteolysis) and in addition, or in the alternative, may arise from differential post-translational modification (*e.g*., glycosylation, acylation, phosphorylation, isoprenylation, lipidation).

"Analog" as used herein, refers to a nucleotide, a protein, or a polypeptide that possesses similar or identical activity or function(s) as the nucleotide, protein or polypeptide having the desired activity and therapeutic effect of the present invention (eg. diagnostic method for determining the predisposition, the onset or the presence of autism spectrum disorder in a mammal), but need not necessarily comprise a sequence that is similar or identical to the sequence of the preferred embodiment, such as that of SEQ ID NOS: 1 and 2, or possess a structure that is similar or identical to that of SEQ ID NOS: 1 and 2. As used herein, a nucleic acid or nucleotide sequence, or an amino acid sequence of a protein or polypeptide is "similar" to that of a nucleic acid, nucleotide or protein or polypeptide having the desired activity if it satisfies at least one of the following criteria: (a) the nucleic acid, nucleotide, protein or polypeptide has a sequence that is at least 30% (more preferably, at least 35%, at least 40%, at least 45%, at least 50%, at least 55%, at least 60%, at least 65%, at least 70%, at least 75%, at least 80%, at least 85%, at least 90%, at least 95% or at least 99%) identical to the nucleic acid, nucleotide, protein or polypeptide sequences having the desired activity as described herein (b) the polypeptide is encoded by a nucleotide sequence that hybridizes under stringent conditions to a nucleotide sequence encoding at least 5 amino acid residues (more preferably, at least 10 amino acid residues, at least 15 amino acid residues, at least 20 amino acid residues, at least 25 amino acid residues, at least 40 amino acid residues, at least 50 amino acid residues, at least 60 amino residues, at least 70 amino acid residues, at least 80 amino acid residue, at least 90 amino acid residues, at least 100 amino acid residues, at least 125 amino acid residues, or at least 150 amino acid residues) of the AAPI; or (c) the polypeptide is encoded by a nucleotide sequence that is at least 30% (more preferably, at least 35%, at least 40%, at least 45%, at least 50%, at least 55%, at least 60%, at least 65%, at least 70%, at least 75%, at least 80%, at least 85%, at least 90%, at least 95% or at least 99%) identical to the nucleotide sequence encoding the polypeptides of the present invention having the desired therapeutic effect. As used herein, a polypeptide with "similar structure" to that of the preferred embodiments of the invention refers to a polypeptide that has a similar secondary, tertiary or quarternary structure as that of the preferred embodiment (eg. SEQ ID NO: 2). The structure of a polypeptide can determined by methods known to those skilled in the art, including but not limited to, X-ray crystallography, nuclear magnetic resonance, and crystallographic electron microscopy.

"Derivative" refers to either a protein or polypeptide that comprises an amino acid sequence of a parent protein or polypeptide that has been altered by the introduction of amino acid residue substitutions, deletions or additions, or a nucleic acid or nucleotide that has been modified by either introduction of nucleotide substitutions or deletions, additions or mutations. The derivative nucleic acid, nucleotide, protein or polypeptide possesses a similar or identical function as the parent polypeptide.

"Fragment" refers to either a protein or polypeptide comprising an amino acid sequence of at least 5 amino acid residues (preferably, at least 10 amino acid residues, at least 15 amino acid residues, at least 20 amino acid residues, at least 25 amino acid residues, at least 40 amino acid residues, at least 50 amino acid residues, at least 60 amino residues, at least 70 amino acid residues, at least 80 amino acid residues, at least 90 amino acid residues, at least 100 amino acid residues, at least 125 amino acid residues, at least 150 amino acid residues, at least 175 amino acid residues, at least 200 amino acid residues, or at least 250 amino acid residues) of the amino acid sequence of a parent protein or polypeptide, or a nucleic acid comprising a nucleotide sequence of at least 10 base pairs (preferably at least 20 base pairs, at least 30 base pairs, at least 40 base pairs, at least 50 base pairs, at least 50 base pairs, at least 100 base pairs, at least 200 base pairs) of the nucleotide sequence of the parent nucleic acid. Any given fragment may or may not possess a functional activity of the parent nucleic acid or protein or polypeptide.

"Modulate" as used herein, refers to a compound or agent (including but not limited to proteins, polypeptides, or fragments thereof, nucleotides, nucleic acid fragments, synthetic organic compounds, antibodies) which are capable of increasing or decreasing the level and/or activity of a gene or gene product identified by the methods described herein, said genes or gene products having a beneficial effect in treating autism and/or Asperger's and/or PDD-NOS. Those skilled in the art, based on the present description, will understand that such modulation can be determined by assays and techniques known to those of skill in the art, including as described in more detail herein.

"Diagnosis" refers to diagnosis, prognosis, monitoring, characterizing, selecting patients, including participants in clinical trials, and identifying patients at risk for or having a particular disorder or clinical event or those most likely to respond to a particular therapeutic treatment, or for assessing or monitoring a patient's response to a particular therapeutic treatment.

As used herein, "detecting a target nucleic acid sequence" refers to determining the presence of a particular target nucleic acid sequence in a sample or determining the amount of a particular target nucleic acid sequence in a sample as an indication of the presence of a target nucleic acid sequence in a sample. The amount of a target nucleic acid sequence that can be measured or detected is preferably about 1 molecule to 10²⁰ molecules, more preferably about 100 molecules to 10¹⁷ molecules and most preferably about 1000 molecules to 10¹⁴ molecules. Preferably there is a direct correlation between the amount of the target nucleic acid sequence and the signal generated by the detected nucleic acid.

As used herein, an "oligonucleotide primer" refers to a single stranded DNA or RNA molecule that is hybridizable (eg. capable of annealing) to a nucleic acid template and is capable of priming enzymatic synthesis of a second nucleic acid strand. Alternatively, or in addition, oligonucleotide primers, when labeled directly or indirectly (e.g., bound by a labeled secondary probe which is specific for the oligonucleotide primer) may be used effectively as probes to detect the presence of a specific nucleic acid in a sample. Oligonucleotide primers useful herein are between about 10 to 100 nucleotides in length, preferably about 17-50 nucleotides in length and more preferably about 17-40 nucleotides in length and more preferably about 17-30 nucleotides in length. Oligonucleotide probes useful for the formation of a cleavage structure are between about 17-40 nucleotides in length, preferably about 17-30 nucleotides in length and more preferably about 17-25 nucleotides in length. The term "primer" may refer to more than one primer and generally refers to an oligonucleotide, whether occurring naturally, as in a purified restriction digest, or produced synthetically, which is capable of acting as a point of initiation of DNA synthesis when annealed to a nucleic acid template and placed under conditions in which synthesis of a primer extension product which is complementary to the template is catalyzed. Such conditions include the presence of four different deoxyribonucleoside triphosphates and a polymerization-inducing agent such as a DNA polymerase or reverse transcriptase, in a suitable buffer ("buffer" includes substituents which are cofactors, or which affect pH, ionic strength, etc.), and at a suitable temperature. The primer is preferably single-stranded for maximum efficiency in amplification.

As used herein, "amplifying" refers to the generation of additional copies of a nucleic acid sequence. A variety of methods have been developed to amplify nucleic acid sequences, including the polymerase chain reaction (PCR). PCR amplification of a nucleic acid sequence generally results in the exponential amplification of a nucleic acid sequence(s) and or fragments thereof. An "amplified" nucleic acid molecule (or portion thereof) refers to the fact that multiple copies of that molecule or a molecule of complementary base sequence have been made.

By "homologous" is meant a same sense nucleic acid which possesses a level of similarity with the target nucleic acid within reason and within standards known and accepted in the art. With regard to PCR, the term "homologous" may be used to refer to an amplicon that exhibits a high level of nucleic acid similarity to another nucleic acid, e.g., the template cDNA. As is understood in the art, enzymatic transcription has measurable and well known error rates (depending on the specific enzyme used), thus within the limits of transcriptional accuracy using the modes described herein, in that a skilled practitioner would understand that fidelity of enzymatic complementary strand synthesis is not absolute and that the amplified nucleic acid (i.e., amplicon) need not be completely identical in every nucleotide to the template nucleic acid.

"Complementary" is understood in its recognized meaning as identifying a nucleotide in one sequence that hybridizes (anneals) to a nucleotide in another sequence according to the rule A→T, U and C→G (and vice versa) and thus "matches" its partner for purposes of this definition. Enzymatic transcription has measurable and well known error rates (depending on the specific enzyme used), thus within the limits of transcriptional accuracy using the modes described herein, in that a skilled practitioner would understand that fidelity of enzymatic complementary strand synthesis is not absolute and that the amplicon need not be completely matched in every nucleotide to the target or template RNA.

As used herein, the terms "nucleic acid", "polynucleotide" and "oligonucleotide" refer to primers, probes, and oligomer fragments to be detected, and shall be generic to polydeoxyribonucleotides (containing 2-deoxy-D-ribose), to polyribonucleotides (containing D-ribose), and to any other type of polynucleotide which is an N-glycoside of a purine or pyrimidine base, or modified purine or pyrimidine bases (including abasic sites). There is no intended distinction in length between the term "nucleic acid", "polynucleotide" and "oligonucleotide", and these terms will be used interchangeably. These terms refer only to the primary structure of the molecule. Thus, these terms include double- and single-stranded DNA, as well as double- and single-stranded RNA.

The "polymerase chain reaction (*PCR*)" technique, is disclosed in U.S. Pat. Nos. 4,683,202, 4,683,195 and 4,800,159. In its simplest form, *PCR* is an in vitro method for the enzymatic synthesis of specific DNA sequences, using two oligonucleotide primers that hybridize to opposite strands and flank the region of interest in the target DNA. A repetitive series of reaction steps involving template denaturation, primer annealing and the extension of the annealed primers by DNA polymerase results in the exponential accumulation of a specific fragment (i.e, an amplicon) whose termini are defined by the 5' ends of the primers. *PCR* is reported to be capable of producing a selective enrichment of a specific DNA sequence by a factor of 109. The *PCR* method is also described in Saiki et al., 1985, Science, 230:1350.

As used herein, "probe" refers to a labeled oligonucleotide primer, which forms a duplex structure with a sequence in the target nucleic acid, due to complementarity of at least one sequence in the probe with a sequence in the target region. Such probes are useful for identification of a target nucleic acid sequence for EN2 Pairs of single-stranded DNA primers can be annealed to sequences within a target nucleic acid sequence or can be used to prime DNA synthesis of a target nucleic acid sequence.

By "genetic abnormality" is meant any change in a nucleic acid sequence which may predispose a person to autism, or Asperger's or PDD-NOS. The change is determined using standard techniques in the art and a comparison is made to a known sequence obtained from a normal individual known to be free of autism, Asperger's or PDD-NOS. Such changes may include mutations and may be deletions, additions or substitutions in at least one base pair of the sequence.

kD stands for kilodalton.

Code-wise degenerate means that a base of a codon is changed without changing the amino acid that the codon codes for.

A "coding sequence" or a "coding region" of a nucleic acid for a designated protein refers to a region in an mRNA molecule that contains the base sequence which is translated into an amino acid sequence (i.e., that encodes that amino acid sequence), it covers any DNA of RNA sequence that is complementary in base sequence to such an mRNA sequence, it covers any DNA sequence that is the same as such an mRNA sequence (except that T is used in place of U) and, in the case of a DNA sequence that alternates introns with exons so that the sequence can be processed to make an mRNA molecule, "coding sequence" or "coding region" covers the populations of coding exons that determine the amino acid coding region of the mRNA molecule (i.e., that encode that amino acid sequence).

By way of example and not limitation, procedures using such conditions of low stringency are as follows (*see also* Shilo and Weinberg, 1981, Proc. Natl. Acad. Sci. U.S.A. 78, 6789-6792). Filters containing DNA are pretreated for 6 h at 40°C in a solution containing 35% formamide, 5X SSC, 50 mM Tris-HCl (pH 7.5), 5 mM EDTA, 0.1% PVP, 0.1% Ficoll, 1% BSA, and 500 µg/ml denatured salmon sperm DNA. Hybridizations are carried out in the same solution with the following modifications: 0.02% PVP, 0.02% Ficoll, 0.2% BSA, 100 µg/ml salmon sperm DNA, 10% (wt/vol) dextran sulfate, and 5-20 X 10⁶ cpm ³²P-labeled probe is used. Filters are incubated in hybridization mixture for 18-20 h at 40°C, and then washed for 1.5 h at 55□C in a solution containing 2X SSC, 25 mM Tris-HCl (pH 7.4), 5 mM EDTA, and 0.1% SDS. The wash solution is replaced with fresh solution and incubated an additional 1.5 h at 60°C. Filters are blotted dry and exposed for autoradiography. If necessary, filters are washed for a third time at 65-68°C and re-exposed to film. Other conditions of low stringency that may be used are well known in the art (*e*.*g*., as employed for cross-species hybridizations).

Procedures using such conditions of moderate stringency are as follows: filters comprising immobilized DNA are pretreated for 6 hours at 55°C in a solution containing 6X SSC, 5X Denhardt's solution, 0.5% SDS and 100 µg/ml denatured salmon sperm DNA. Hybridizations are carried out in the same solution with 5-20 x 10⁶ cpm ³²P-labeled probe. Filters are incubated in hybridization mixture for 18-20 hours at 55°C, and then washed twice for 30 minutes at 60°C in a solution containing 1X SSC and 0.1% SDS. Filters are blotted dry and exposed for autoradiography. Washing of filters is done at 37°C for 1 hour in a solution containing 2X SSC, 0.1% SDS. Other conditions of moderate stringency that may be used are well known in the art. (*see, e*.*g*., Sambrook et al., 1989, Molecular Cloning, A Laboratory Manual, 2d Ed., Cold Spring Harbor Laboratory Press, Cold Spring Harbor, New York; *see also*, Ausubel et al., eds., in the Current Protocols in Molecular Biology series of laboratory technique manuals, 1987-1997 Current Protocols,© 1994-1997 John Wiley and Sons, Inc.).

Procedures using such conditions of high stringency are as follows. Prehybridization of filters containing DNA is carried out for 8 h to overnight at 65°C in buffer composed of 6X SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 48 h at 65°C in prehybridization mixture containing 100 µg/ml denatured salmon sperm DNA and 5-20 X 10⁶ cpm of ³²P-labeled probe. Washing of filters is done at 37°C for 1 h in a solution containing 2X SSC, 0.01% PVP, 0.01% Ficoll, and 0.01% BSA. This is followed by a wash in 0.1X SSC at 50°C for 45 min before autoradiography. Other conditions of high stringency that may be used are well known in the art.

An RNA base sequence (or molecule) is equivalent to a DNA base sequence (or molecule) if they are identical except that U in the RNA base sequence replaced T in the DNA base sequence.

The following one letter codes are used to represent amino acids:
S-serine, T-threonine, N-asparagine, Q-glutamine, K-lysine, R-arginine,
H-histidine, E-glutamic acid, D-aspartic acid, C-cystine, G-glycine,
P-proline, A-alanine, I-isoleucine, L-leucine, M-methionine,
F-phenylalanine, W-tryptophan, V-valine, Y-tyrosine, X-any amino acid.

The following one letter codes are used to represent nucleic acids:
A-adenine, C-cytosine, G-guanine, T-thymidine, R represents A or G, Y
represents T or C, N represents any nucleic acid.

An EN2 nucleic acid or protein referred to herein is a human one unless otherwise noted.

A chromosome region is spanned by two sequences if one sequence is at one end of the region and the other sequence is at the other end of the region.

An EN2 protein includes any protein or fragment encoded by the EN2 gene, including any segment, region, or exon.

### General Description

Autism and allied autistic spectrum (AS) disorders present myriad behavioral, clinical, and biochemical abnormalities. Several studies have demonstrated that AS has a genetic basis. Family studies have determined that there is a 75x greater chance of siblings inheriting the disorder than the general population if a brother or sister already has autism (Bolton et al (1994), J Child Psychol Psychiat 35: 877-900). Monozygotic twin studies display a 75% concordance in symptoms in comparison to only 10% in fraternal twins (Bailey et al (1998), Brain 121:889-905, Folstein and Rutter (1977), J Child Psychol Psychiat 18: 297-321, Ritvo et al (1985), Am. J. Psychiat 142: 74-77). This large difference suggests that multiple loci are involved, with some models predicting more than 15 genes contributing to the disorder (Folstein and Rosen-Sheidley (2001), Nat Rev Genet 2:943-955, Lamb et al (2000) Hum Molec Genet 9: 861-868, Risch et al (1999), Am J Hum Genet 65: 493-507).

Furthermore, the cerebellum is most often malformed in autistic individuals. 21/22 autopsy studies have revealed cerebellar abnormalities including Purkinje cell loss. These defects occur in the absence of any obvious sign of degeneration suggesting that autism is instead caused by developmental defects (Bailey et al (1998) Brain 121:889-905, Bauman and Kemper (1985), Neurology 35: 866-874, Bauman and Kemper (1986), Neurology 36(suppl. 1): 190, Bauman and Kemper (1994) In The Neurobiology of Autism. Baltimore: John Hopkins University Press pp119-145, Courchesne (1997), Learn Mem 4:1-35, Kemper and Bauman (1993), Behav Neuro 11: 175-187, Ritvo et al (1996) Am J Psychiatry 143:862-866). In addition, imaging studies have also demonstrated cerebellar hypoplasia (Courchesne et al (1988), New Eng J Med 318: 1349-1354, Courchesne (1997) Curr Opin Neurobiol 7:269-278, Gaffney et al (1987), Br J Psychiatry 151:831-833, Hashimoto et al (1995), J Autism Dev Discord 25:1-18, Kleiman et al (1992), Neurology 42:753-760, Murakami et al (1989), Arch Neurol 46:689-694). A recent imaging report has uncovered abnormal cerebellar growth patterns after birth. The growth is initially accelerated followed by a decrease after age six (Courchesne et al (2001), Neurology 57:245-254). These studies indicate that cerebellar development is perturbed in autism. Interestingly, recent fMRI studies indicate that the cerebellum is active during tasks that are defective in autism including language and attention (Akshoomoff et al (1997), Int Rev Neurobiol 41:575-98, Allen et al (1997), Science 275:1940-1943, Allen and Courchesne (2003), Am J Psychiatry 160:262-73, Courchesne et al (1994), Behavioral Neuroscience 108:848-865, Courchesne and Allen (1997), Learn Mem 4:1-35, Gao et al (1996), Science 272:545-547). Thus, these anatomical defects might directly contribute to the behavioral abnormalities associated with autism.

Mouse genetics have identified a large number of genes that function during cerebellar development (Hatten and Heintz (1995), Annu Rev Neurosci 18:385-408, Hatten et al. (1997), Curr Opin Neurobiol 7(1):40-47). One such gene is *ENGRAILED 22*, a homeobox transcription factor that is orthologous to Drosophila melanogaster *ENGRAILED 2*. In the mouse, there are two *ENGRAILED 2* genes, *En1* and *En2*. Knockout studies have demonstrated that *En1* functions during early A-P patterning of neural tube while *En2* is specifically required for normal postnatal cerebellar development (Millen et al. (1994), Development 120:695-706; Hanks et al. (1995), Science 269:679-682; Millen et al. (1995), Development 121:3935-45; Liu and Joyner (2001), Annu Rev Neurosci 24:869-896 ). The *En2*^{-/-} mutant displays cerebellar hypoplasia that is due in part to a reduction in all the major cerebellar cell types including Purkinje cells (Millen et al. (1994), Development 120:695-706; Millen et al. (1995), Development 121:3935-45; Kuemerle et al. (1997), J Neurosci 17:7881-9). Interestingly, human *EN2* maps to distal chromosome 7, a region that displays linkage to AS in two soparate studies (Liu et al. (2001), Annu Rev Neurosci 24:869-896, Auranen et al. (2002), Am J Hum Genet 71:777-790). For these reasons, *EN2* was tested as a susceptibility locus for AS by performing family based association aualysis.

Research suggests that early diagnosis of autism or autism related disorders such as Asperger's or PDD-NOS may be associated with dramatically better outcomes. The earlier a child is diagnosed, the earlier the child may begin benefiting from one of the many specialized intervention approaches.

Because a behaviour-based diagnosis of autism is difficult, especially in young patients, it would be valuable to have a genetic screening assay to assist in the diagnosis. Moreover, such an assay can be used 10 advise potential parents of their chances of having autistic children.

However, up until the disclosure in the present invention, there has been no genetic marker available which is indicative of a subject's susceptibility to autism, or a disease related thereto, such as Asperger's Disorder or PDD-NOS. Also needed is the development of potential drugs or agents that have applications in treating autism or a disease or disorder related thereto, such as Asperger's Disorder or PDD-NOS.

There is described an isolated nucleic acid molecule (SEQ ID NO:1), which encodes human EN2, and the amino acid sequence of human EN2 (SEQ ID NO: 2). The position of EN2 on chromosome 7 is between position 154552000 and position 154560000. More particularly, there is described methods for determining a subject's susceptibility to autism or a disease or disorder related thereto, such as Asperger's Disorder or PDD-NOS using a variant allele of the *ENGRAILED* 2 *(En2)* gene, which maps to chromosome 7, in particular, to 7q36.3. Furthermore, transmission/disequilibrium tests (TDT), which were performed for two single nucleotide polymorphisms (*rs1861972* and *rs1861973*), revealed significant overtransmission of the A allele of *rs1861972* and the C allele of *rs1861973* (*rs1861972 P=*0.0009; rs1861973 *P*=0.0006). The *rs1861972* SNP, which is located at position 154670848, results in a guanine to adenine transition, whereas *rs1861973,* located at position 154671000, results in a thymine to cytosine transition. Haplotype analysis indicated that the A, C haplotype is specifically overtransmitted in autistic individuals (*P*=0.000062). Detection of such a variant allele in the genome of a subject may be indicative of the subject's susceptibility to autism. Furthermore, a variant allele of the *ENGRAILED 2* gene can also be used to assay drugs and agents for potential use in treating autism or a disease or disorder related thereto, such as Asperger's Disorder or PDD-NOS.

The identification of particular single nucleotide polymorphisms (SNPs), which show statistical relevance in terms of being markers of susceptibility to autism and autism related disorders is described. In particular, the two SNPs have been found on chromosome 7, in the gene that encodes for the transcription factor EN2.

In particular, the homeobox transcription factor, *ENGRAILED 2* (*EN2*), was investigated for association with autism spectrum disorder (AS) by performing transmission/disequilibrium tests (TDT) for two SNPs (*rs1861972* and *rs1861973*). Initially, TDT was performed using 137 triads of autistic individuals and their parents and significant overtransmission of the A allele of *rs1861972* and the C allele of *rs1861973* was observed (*rs1861972 P*=0.0009; *rs1861973 P*=0.0006; A,C haplotype *P*=0.000062). The analysis was then extended to include other affected and unaffected siblings as well as 29 additional families. Significant association is observed for both SNPs under both the broad and narrow diagnostic schemes *(rs1861972*: narrow *P*=0.0106, broad *P*=0.0050; *rs81861973:* narrow *P*=0.0033, broad *P*=0.0063; A,C Haplotype: narrow *P*=0.0007, broad *P*=0.0009). The procedures utilized for the analyses in these studies are known to those skilled in the art (Martin, E. R et al, (2000), Am. J. Hum. Genet. 67: 146-154; Terwilliger, J.D., (1995), Am. J. Hum. Genet. 56:777-787; Ye, S. et al., (2001). Nucleic Acids Res. 29: E88-8; Clayton, D. (1999), Am. J. Hum. Genet. 65:1170-1177; Strachan, T. and Read, A., Human Molecular Genetics 2, Wiley Liss, New York, NY (1999)). In summary, these results identify *EN2* as a likely susceptibility locus for autism and related AS disorders.

### Current Diagnostic Criteria for Identification of Autism and Asperger's Disorder

The characteristic behaviors of autism spectrum disorders may or may not be apparent in infancy (18 to 24 months), but usually become obvious during early childhood (24 months to 6 years).

While there is no one behavioral or communications test that can detect autism, several screening instruments have been developed that are now used in diagnosing autism.

CARS rating system (Childhood Autism Rating Scale), developed by Eric Schopler in the early 1970s, is based on observed behavior. Using a 15-point scale, professionals evaluate a child's relationship to people, body use, adaptation to change, listening response, and verbal communication.

The Checklist for Autism in Toddlers (CHAT) is used to screen for autism at 18 months of age. It was developed by Simon Baron-Cohen in the early 1990s to see if autism could be detected in children as young as 18 months. The screening tool uses a short questionnaire with two sections, one prepared by the parents, the other by the child's family doctor or pediatrician.

The Autism Screening Questionnaire is a 40 item screening scale that has been used with children four and older to help evaluate communication skills and social functioning.

The Screening Test for Autism in Two-Year Olds, being developed by Wendy Stone at Vanderbilt, uses direct observations to study behavioral features in children under two. She has identified three skills areas - play, motor imitation, and joint attention - that seem to indicate autism.

The diagnostic criteria for Asperger's Disorder are as follows:
A qualitative impairment in social interaction, as manifested by at least two of the following:
   1. marked impairments in the use of multiple nonverbal behaviors such as eye-to-eye gaze, facial expression, body postures, and gestures to regulate social interaction
   2. failure to develop peer relationships appropriate to developmental level
   3. a lack of spontaneous seeking to share enjoyment, interests, or achievements with other people (e.g. by a lack of showing, bringing, or pointing out objects of interest to other people)
   4. lack of social or emotional reciprocity

Restricted repetitive and stereotyped patterns of behavior, interests, and activities, as manifested by at least one of the following:
1. encompassing preoccupation with one or more stereotyped and restricted patterns of interest that is abnormal either in intensity or focus
2. apparently inflexible adherence to specific, nonfunctional routines or rituals
3. stereotyped and repetitive motor mannerisms (e.g., hand or finger flapping or twisting, or complex whole-body movements)
4. persistent preoccupation with parts of objects

The disturbance causes clinically significant impairment in social, occupational, or other important areas of functioning

There is no clinically significant general delay in language (e.g., single words used by age 2 years, communicative phrases used by age 3 years)

There is no clinically significant delay in cognitive development or in the development of age-appropriate self-help skills, adaptive behavior (other than social interaction), and curiosity about the environment in childhood

Criteria are not met for another specific Pervasive Developmental Disorder or Schizophrenia

Because a behaviour-based diagnosis of autism is difficult, especially in young patients, it would be valuable to have a genetic screening assay to assist in the diagnosis. Furthermore, such an assay can be used to advise potential parents of their chances of having autistic children.

However, up until the disclosure in the present invention, there has been no genetic marker available which is indicative of a subject's susceptibility to autism, or a disease related thereto, such as Asperger's Disorder or PDD-NOS.

### Obtaining proteins

EN2 protein for use herein can be isolated and purified according to techniques known in the art.

The protein may be made by known methods of chemical synthesis, or expressed. The description below provides examples of materials and methods for so doing, but is not intended to be either limiting or exclusive.

### Expression of Proteins

The nucleotide sequence encoding the protein (provided herein) or antigenic fragment, derivative or analog thereof, or a functionally active derivative, including a chimeric protein, thereof, can be inserted into an appropriate expression vector, *i*,*e*, a vector which contains the necessary elements for the transcription and translation of the inserted protein-coding sequence. Such elements are termed herein a "promoter." Thus, the nucleic acid encoding the EN2 Protein of the protein described herein is operationally associated with a promoter in an expression vector. Both cDNA and genomic sequences can be cloned and expressed under control of such regulatory sequences. An expression vector also preferably includes a replication origin.

The necessary transcriptional and translational signals can be provided on a recombinant expression vector, or they may be supplied by the gene encoding EN2 protein and/or its flanking regions. Chimeric proteins including EN2 protein may also be Produced as described.

Potential host-vector systems include but are not limited to mammalian cell systems infected with virus (*e*.*g*., vaccinia virus, adenovirus, etc.); insect cell systems infected with virus (*e*.*g*., baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

In this regard, any of a number of amplification systems may be used to achieve high levels of stable gene expression.

The cell containing the recombinant expression vector is cultured in an appropriate cell culture medium under conditions that provide for expression of protein by the cell.

Any of the methods previously described for the insertion of DNA fragments into a cloning vector may be used to construct expression vectors containing a gene consisting of appropriate transcriptional/translational control signals and the protein coding sequences. These methods may include *in vitro* recombinant DNA and synthetic techniques and *in vivo* recombination (genetic recombination).

Expression may be controlled by any promoter/enhancer element known in the art, but these regulatory elements must be functional in the host selected for expression. Promoters which may be used to control EN2 gene expression include, but are not limited to, the SV40 early promoter region (Benoist and Chambon, Nature, 290:304-310 (1981)), the promoter contained in the 3N long terminal repeat of Rous sarcoma vitus (Yamamoto, et al., Cell, 22:787-797 (1980)), the herpes thymidine kinase promoter (Wagner et al., Proc. Natl. Acad. Sci. U.S.A, 78:1441-1445 (1981)), the regulatory sequences of the metallothionein gene (Brinster et al., Nature, 296:39-42 (1982)); prokaryotic expression vectors such as the ∃-lactamase promoter (Villa-Kamaroff, et al., Proc. Natl. Acad. Sci. U.S.A., 75:3727-3731 (1978)), or the *tac* promoter (DeBoer, et al., Proc. Natl. Acad. Sci. U.S.A., 80:21-25 (1983)); see also " Scientific American, 242:74-94 (1980); promoter elements from yeast or other fungi such as the Gal 4 promoter, the ADC (alcohol dehydrogenase) promoter, PGK (phosphoglycerol kinase) promoter, alkaline phosphatase promoter; and the animal transcriptional control regions, which exhibit tissue specificity and have been utilized in transgenic animals: elastase I gene control region which is active in pancreatic acinar cells (Swift et al., Cell, 38:639-646 (1984)); (Omitz et al., Cold Spring Harbor Symp. Quant. Biol., 50:399-409 (1986)); (MacDonald, Hepatology, 7:425-515 (1987)); insulin gene control region which is active in pancreatic beta cells (Hanahan. Nature, 315:115-122 (1985)), immunoglobulin gene control region which is active in lymphoid cells (Crosschedl et al., Cell, 38:647-658 (1984)); (Adames et al., Nature, 318:533-538 (1985)); (Alexander et al., Mol. Cell. Biol., 7:1436-1444 (1987)), mouse mammary tumor virus control region which is active in testicular, breast, lymphoid and mast cells (Leder et al., Cell, 45:485-495 (1986)), albumin gene control region which is active in liver (Pinkert et al., Genes and Devel., 1:268-276 (1987)), alpha-fetoprotein gene control region which is active in liver (Krumlauf et al., Mol. Cell. Biol., 5:1639-1648 (1985)); (Hammer et al., Science, 235:53-58 (1987)), alpha 1-antirypsin gene control region which is active in the liver (Kelsey et al., Genes and Devel., 1:161-171 (1987)), beta-globin gene control region which is active in myeloid cells (Mogram et al., Nature, 315:338-340 (1985)); (Kollin et al., Cell, 46:89-94 (1986)), myelin basic protein gene control region which is active in oligodendrocyte cells in the brain (Readhead et al., Cell, 48:703-712 (1987)), myosin light chain-2 gene control region which is active in skeletal muscle (Sani, Nature, 314:283-286 (1985)), and gonadotropic releasing hormone gene control region which is active in the hypothalamus (Mason et al., Science, 234:1372-1378 (1986)).

Expression vectors containing a nucleic acid encoding an EN2 protein can be identified by four well known approaches: (a) PCR amplification of the desired plasmid DNA or specific mRNA, (b) nucleic acid hybridization, (c) presence or absence of selection marker gene functions, and (d) expression of inserted sequences.

A wide variety of host/expression vector combinations may employed in expressing the EN2 gene or fragments. Useful expression vectors, for example, may consist of segments of chromosomal, non-chromosomal, and synthetic DNA sequences. Suitable vectors include derivatives of SV40 and known bacterial plasmids, e.g., *E*. *coli* plasmids col E1, pCR1, pBR322, pMal-C2, pET, pGEX (Smith et al., Gene, 67:31-40 (1988)), pMB9 and their derivatives, plasmids such as RP4; phage DNAS, e.g., the numerous derivatives of phage 8, e.g., NM989, and other phage DNA, e.g., M13 and filamentous single stranded phage DNA; yeast plasmids such as the 2: plasmid or derivatives thereof; vectors useful in eukaryotic cells, such as vectors useful in insect or mammalian cells; vectors derived form combinations of plasmids and phage DNAs, such as plasmids that have been modified to employ phage DNA or other expression control sequences; and the like.

For example, in a baculovirus expression systems, both non-fusion transfer vectors, such as but not limited to pVL941 (Summers), pVL1393 (Invitrogen), pVL1392 (Summers and Invitrogen), and pBlue*Bac*III (Invitrogen), and fusion transfer vectors, such as but not limited to pAc700 (Summers), pAc701 and pAc702, pAc360 (Invitrogen), and pBlueBacHisA, B, C (Invitrogen).

Mammalian expression vectors contemplated for use include vectors with inducible promoters, such as the dihydrofolate reductase (DHFR) promoter, *e.g.,* any expression vector with a *DHFR* expression vector, or a *DHFR*/methotrexate co-amplification vector, such as pED (*Pst*I, *Sal*I, *Sba*I, *Sma*I, and *ECO*RI cloning site, with the vector expressing both the cloned gene and *DHFR*; *see* Kaufman, Current Protocols in Molecular Bilogy, 16.12 (1991). Alternatively, a glutamine synthetase/methionine sulfoximine co-amplification vector, such as pEE14 (Celltech). A vector that directs episomal expression under control of Epstein Barr Virus (BBV) can be und, such as pREP4 (Invitrogen), pCBP4 (Invitrogen), pMBP4 (Invitrogen), pREP8 (Invitrogen), pREP9 (Invitrogen), and pEBVHis (Invitrogen). Selectable mammalian expression vectors include pRc/CMV (Invitnogen), pRc/RSV (Invitrogen), and others. Vaccinia virus mammalian expression vectors (*see*, Kaufman, 1991, *supra*) include but are not limited to pSC11 and pMJ601.

Yeast expression systems can also be used. For example, the non-fusion pYEN2 vector (Invitrogen) or the fusion pYESHisA, B, C (Invitrogen), to mention just two, can employed.

Once a particular recombinant DNA molecule is identified and isolated, several methods known in the art may be used to propagate it. Once a suitable host system and growth conditions are established, recombinant expression vectors can be propagated and prepared in quantity. As previously explained, the expression vectors which can be used include, but are not limited to, the following vectors or their derivatives: human or animal viruses such as vaccinia virus or adenovirus; insect viruses such as baculovirus; yeast vectors; bacteriophage vectors (*e*.*g*.,Iambda), and plasmid and cosmid DNA vectors, to mane but a few.

In addition, a host cell strain may be chosen which modulates the expression of the inserted sequences, or modifies ad processes the gene product in the specific fashion desired. Different host cells have characteristic and specific mechanisms for the translational and post-translational processing and modification (*e.g*., glycosylation, cleavage *e*.*g*., of signal sequence) of proteins. Appropriate cell lines or host systems can be chosen to ensure the desired modification and processing of the foreign protein expressed. For example, expression in a bacterial system can be used to produce an non-glycosylated core protein product.

Vectors are introduced into the desired host cells by methods known in the art, *e*.*g*., transfection, electroporation, micro injection, transduction, cell fusion, DEAE dextran, calcium phosphate precipitation, lipofection (lysosome fusion), use of a gene gun, or a DNA vector transporter (see, *e.g*., Wu et al., J. Biol. Chem., 267:963-967 (1992); Wu and Wu, J. Blol. Chem., 263:14621-14624 (1988); Hartmut *et al*., Canadian Patent Application No. 2,012,311, filed March 15,1990).

### Purification of Proteins

The purification of EN2 Protein can be accomplished by any number of procedures that encompass a wide variety of known purification steps. Those with skill in the art would know to refer to references, such as the Methods of Enzymology series, for greater detail and breadth. Initial steps for purifying the proteins include salting in or salting out, such as in ammonium sulfate fractionations; solvent exclusion fractionations, *e*.*g*., an ethanol precipitation; detergent extractions to free membrane bound proteins using such detergents as Triton X-100, Tween-20 etc.; or high salt extractions. Solubilization of proteins may also be achieved using aprotic solvent such as dimethyl sulfoxide and hexamethylphosphoramide. In addition, high speed ultracentrifugation may be used either alone or in conjunction with other extraction techniques. A useful method is tagging the protein with hexa-histidine via Ni+ chromatography (Coligan, et al. Current Protocols in Protein Science, vol. 1 (John Wiley and Sons, New York, 1995); Methods in Enzymology (M.P. Deutscher, ed.) vol. 185 Guide to Protein Purification (Academic Press, San Diego, California 1990); Crowe, et al. 1994 Methods Mol. Biol. 31:371-387); Schmitt, et al. 1993 Molecular Biology Reports 18:223-230).

Generally good secondary isolation or purification steps include solid phase absorption using calcium phosphate gel or hydroxyapatite; or solid phase binding. Solid phase binding may be performed through ionic bonding, with either an anion exchanger, such as diethylaminoethyl (DEAE), or diethyl [2-hydroxy propyl] amino ethyl (QAE) Sephadex or cellulose; or with a cation exchanger such as carboxymethyl (CM) or sulfo propyl (SP) Sephadex or cellulose. Alternative means of solid phase binding includes the exploitation of hydrophobic interactions *e.g.,* the using of a solid support such as phenylSepharose and a high salt buffer; affinity-binding, bound to an activated support; immuno-binding, using *e.g.,* an antibody to the EN2 protein bound to an activated support; as well as other solid phase supports including those that contain specific dyes or lectins etc. A further solid phase support technique that is often used at the end of the purification procedure relies on size exclusion, such as Sephadex and Sepharose gels, or pressurized or centrifugal membrane techniques, using size exclusion membrane filters.

Solid phase support separations are generally performed batch-wise with low-speed centrifugations or by column chromatography. High performance liquid chromatography (HPLC), including such related techniques as FPLC, is presently the most common means of performing liquid chromatography. Size exclusion techniques may also be accomplished with the aid of low speed centrifugation.

In addition size permeation techniques such as gel electrophoretic techniques may be employed. These techniques are generally performed in tubes, slabs or by capillary electrophoresis.

Almost all steps involving protein purification employ a biological buffer at a pH close to the pKa of that buffer. Typical buffers can be purchased from most biochemical catalogues and include the classical buffers such as Tris, pyrophosphate, monophosphate, and diphosphate, or the Good buffers (Good, N.E., et al., Biochemistry, 5:467 (1966); Good, N.E. and Izawa, S., Meth. Enzymol., 24 Part B, 53(1972); and Fergunson, W.J. and Godd, N.E., Anal. Biochem., 104:300 (1980)) such as Mes, Hepes, Mops, tricine and Ches.

Materials to perform all of these techniques are available from a variety of sources such as Sigma Chemical Company in St. Louis, Missouri.

### Obtaining antibodies

Antibodies can be produced according to techniques known in the art. The description below provides examples of materials and methods for so doing, but is not intended to be either limiting or exclusive.

EN2 protein may produced recombinantly or by chemical synthesis as described above, and fragments or other derivatives or analogs thereof, including fusion proteins, may be sued as an immunogen to generate antibodies that recognize the protein. polypeptide. Such antibodies include but are not limited to polyclonal, monoclonal, chimeric, single chain, Fab fragments, and an Fab expression library. The anti-EN2 Protein antibodies may be cross reactive, e*.g*., they may recognize EN2 Protein from different species. Polyclonal antibodies have greater likelihood of cross reactivity. Alternatively, an antibody may be specific for a single form of EN2 protein, such as murine EN2 Protein. Preferably, such as antibody is specific for human BN2 Protein.

Various procedures known in the art may be used for the production of polyclonal antibodies to EN2 Protein. For the production of antibody, various host animals can be immunized by injection with the EN2 Protein, including but not limited to rabbits, mice, rats, sheep, goats, etc. The EN2 protein thereof can be conjugated to an immunogenic carrier, *e*.*g*., bovine serum albumin (BSA) or keyhole limpet hemocyanin (KLH). Various adjuvants may be used to increase the immunological response, depending on the host species, includig but not limited to Freund's (complete and incomplete), mineral gels such as aluminum hydroxide, surface active substances such as lysolecithin, pluronic polyols, polyanions, peptides, oil emulsions, keyhole limpet hemocyanins, dimitrophenol, and potentially useful human adjuvants such as BCG (*bacille Calmette-Guerin*) and *Corynebacterium parvum*.

For preparation of monoclonal antibodies directed toward the EN2 Protein, any technique that provides for the production of antibody molecules by continuous cell lines in culture may bo used. These include but are not limited to the hybridoma technique orginally developed by Kohler and Milstein (Nature 256:495-497 (1975)), as well as the trioma techmique, the human B-cell hybridoma technique (Kozbor et al, Immunology Today 4:72 1983); Cote et al.; Proc. Natl. Acad. Sci. U.S.A. 80:2026-2030 (1983)), and the EBV-hybridoma technique to produce human monoclonal antibodies (Cole et al., in Monoclonal Antibodies and Cancer Therapy, Alan R. Liss, Inc., pp. 77-96 (1985)). Monoclonal antibodies can be produced in germ-free animals utilizing recent technology (PCT/US90/02545). In fact, techniques developed for the production of "chimeric antibodies" (Morrison et al., J. Bacteriol. 159:870 (1984); Neuberger et al., Nature 312:604-608 (1984); Takeda et al., Nature 314:452-454 (1985)) by splicing the genes from a mouse antibody molecule specific for an BN2 Protein together with genes from a human antibody molecule of appropriate biological activity can be used.
Such human or humanized chimeric antibodies are preferred for use in therapy of human diseases or disorders (described *infra*), since the human or humanized antibodies are much less likely than xenogenic antibodies to induce an immune response, in particular an allergic response, themselves.

Techniques described for the porduction of single chain antibodies (U.S. Patent Nos. 5,476,786 and 5,132,405 to Huston; U.S. Patent 4,946,778) can be adapted to produce EN2 Protein-specific single chain antibodies. The techniques described for the construction of Fab expression libraries (Huse et al., Science 246:1275-1281 (1989)) allow rapid and easy identification of monoclonal Fab fragments with the desired specificity for an EN2 Protein. Antibody fragments which contain the idiotype of the antibody molecule can be generated by known techniques. For example, such fragments include but are not limited to: the F(abN)₂ fragment which can be produced by pepsin digestion of the antibody molecule; the FabN fragments which can be generated by reducing the disulfide bridges of the F(abN)₂ fragment, and the Fab fragments which can be generated by treating the antibody molecule with papain and a reducing agent.

In the production of antibodies, screening for the desired antibody can be accomplished by techniques known in the art, *e*.*g*., radioimmunoassay, ELISA (enzyme-linked immunosorbant assay), "sandwich" immunoassays, immunoradiometric assays, gel diffusion precipitin reactions, immunodiffusion assays, *in situ* immunoassay (using colloidal gold, enzyme or radioisotope labels, for example), western blots, precipitation reactions, agglutination assays (*e*.*g*., gel agglutination assays, hemagglutination assays), complement fixation assays, immunofluorescence assays, protein A assays, and immunoelectrophoresis assays, etc. Antibody binding may be detected by detecting a label on the primary antibody, or the primary antibody may be detected by detecting binding of a secondary antibody or reagent to the primary antibody. Also the secondary antibody may be labeled. Many means are known in the art for detecting binding in an immunoassay.
For example, to select antibodies which recognize a specific epitope of an EN2 protein, one may assay generated hybridomas for a product which binds to an EN2 protein fragment containing such epitope. For selection of an antibody specific to an EN2 protein from a particular species of animal, one can select on the basis of positive binding with EN2 protein expressed by or isolated from cells of that species of animal.

The foregoing antibodies can be used in methods known in the art relating to the localization and activity of the EN2 protein, *e*.*g*., for Western blotting imaging EN2 protein *in situ*, measuring levels thereof in appropriate physiological samples, etc. using any of the detection techniques mentioned above or known in the art.

In a specific example, antibodies that agonize or antagonize the activity of EN2 Protein can be generated. Such antibodies can be tested using the assays described *infra* for identifying ligands.

In an immunoassay, a control quantity of the antagonists or antibodies thereto, or the like may be prepared and labeled with an enzyme, a specific binding partner and/or a radioactive element, and may then be introduced into a cellular sample. After the labeled material or its binding partner(s) has had an opportunity to react with sites within the sample, the resulting mass may be examined by known techniques, which may vary with the nature of the label attached.

In the instance where a radioactive label, such as the isotopes ³H, ¹⁴C, ³²P, ³⁵S, ³⁶Cl, ⁵¹Cr, ⁵⁷Co, ⁵⁸Co,⁵⁹Fe, ⁹⁰Y, ¹²⁵I, ¹³¹I, and ¹⁸⁶Re are used, known currently available counting procedures may be utilized. In the instance where the label is an enzyme, detection may be accomplished by may of the presently utilized colorimetric, spectrophotmetric, fluorospectrophotometric, amperometric or gasometric techniques known in the art.

An assay system which may be prepared in the form of a test kit for the quantitative analysis of the extent of the presence of the EN2 protein, or to identify drugs or other agents that may mimic or block its activity is described. Alternatively, the presence of the single nucleotide polymorphisms can be monitored using standard techniques known in the art. The system or test kit may comprise a labeled component prepared by one of the radioactive and/or enzymatic techniques discussed herein, coupling a label to the antibody or an agonist and/or antagonist, and one or more additional immunochemical reagents, at least one of which is a free or immobilized ligand, capable either of binding with the labeled component, its binding partner, one of the components to be determined or their binding partner(a).

### Administration of cells, genes, and proteins to a person

Gene therapy: The EN2 gene or appropriate fragments of the gene (for example corresponding to a deleted region in an individual patient) can be used in gene therapy by methods known in the art. For example, the gene may be introduced *in vivo* in a viral vector. Such vectors include an attenuated or defective DNA virus, such as but not limited to herpes simplex virus (HSV), papillomavirus, Epstein Barr virus (EBV), adenovirus, adeno-associated virus (AAV), and the like. Defective viruses, which entirely or almost entirely lack viral genes, are preferred. Defective virus is not infective after introduction into a cell. Use of defective viral vectors allows for administration to cells in a specific, localized area, without concern that the vector can infect other cells. Examples of particular vectors, include, but are not limited to, a defective herpes virus 1 (HSV1) vector (Kaplitt et al., Molec. Cell. Neurosci. 2:320-330 (1991)), an attenuated adenovirus vector, such as the vector described by Stratford-Perricaudet et al. (J. Clin. Invest. 90:626-630 (1992)), and a defective adeno-associated virus vector (Samulski et al., J. Virol. 61:3096-3101 (1987); Samulski et al., J. Virol. 63:3822-3828 (1989)).

Preferably, for *in vitro* administration, an appropriate immunosuppressive treatment is employed in conjunction with the viral vector, *e*.*g*., adenovirus vector, to avoid immuno-deactivation of the viral vector and transfected cells. For example, immunosuppressive cytokines, such as interleukin-12 (IL-12), interferon-((IFN-(), or anti-CD4 antibody, can be administered to block humoral or cellular immune responses to the viral vectors (*see, e.g*., Wilson, Nature Medicine (1995))*.* In addition, it is advantageous to employ a viral vector that is engineered to express a minimal number of antigens.

In another example the gene can be introduced in a retroviral vector, *e*.*g*., as described in Anderson et al., U.S. Patent No. 5,399,346; Mann et al., 1983, Cell 33:153; Temin et al., U.S. Patent No. 4,650,764; Temin et aL, U.S. Patent No. 4,980,289; Markowitz et al., 1988, J. Virol. 62:1120; Temin et al., U.S. Patent No. 5,124,263; International Patent Publication No. WO 95/07358, published March 16, 1995, by Dougherty et al.; and Kno et al., 1993, Blood 82:845. Targeted gene delivery is described in International Patent Publication WO 95/28494, published October 1995.

Alternatively, the vector can be introduced *in vivo* by lipofection. Synthetic cationic lipids can be used to prepare liposomes for *in vivo* transfection of a gene encoding a marker (Felgner, et. al., Proc. Natl. Acad. Sci. U.S.A. 84:7413-7417 (1987); *see* Mackey, et al., Proc. Natl. Acad. Sci. U.S.A. 85:8027-8031 (1988)). The use of cationic lipids may promote encapsulation of negatively charged nucleic acids, and also promote fusion with negatively charged cell membranes (Felgner and Ringold, Science 337:387-388 (1989)). Lipids may be chemically coupled to other molecules for the purpose of targeting (*see* Mackey, et. al., *supra).* Targeted petides, *e.g.*, hormones or neurotransmitters, and proteins such as antibodies, or non-peptide molecules could be coupled to liposomes chemically.

It is also possible to introduce the vector *in vivo* as a naked DNA plasmid. Naked DNA vectors for gene therapy can be introduced into the desired host cells by methods known in the art, *e.g.*, transfection, electroporation, microinjection, transduction, cell fusion, DEAE dextran, calcium phosphate precipitation, use of a gene gun, or use of a DNA vector transporter (*see*, *e*.*g*., Wu et al., J. Biol. Chem. 267:963-967 (1992); Wu and Wu, J. Biol. Chem. 263:14621-14624 (1988); Hartmut et al., Canadian Patent Application No. 2,012,311, filed March 15, 1990).

A regulatable expression vector is particularly useful to regulate expression of a therapeutic EN2 gene. Constitutive expression of the EN2 gene is contemplated, even if at low levels. Various therapeutic heterologous genes can be inserted in a gene therapy vector such as but not limited to adenosine deaminase (ADA) to treat severe combined immunodeficiency (SCID); marker genes or lymphokine genes into tumor infiltrating (TIL) T cells (Kasis et al., Proc. Natl. Acad. Sci. U.S.A. 87:473 (1990); Culver et aL, ibid. 88:3155 (1991)); genes for clotting factors such as Factor VIII and Factor IX for treating hemophilia (Dwarki et al. Proc. Natl. Acad. Sci. USA, 92:1023-1027 (19950); Thompson, Thromb. and Haemostatis, 66:119-122 (1991)); and various other well known therapeutic genes such as, but not limited to, ∃-globin, dystrophin, insulin, crythropoietin, growth hormone, glucocerebrosidase, ∃-glucuronidase, ∀-antitrypsin, phenylalanine hydroxylase, tyrosine hydroxylase, ornithine transcarbamylase, apolipoproteins, and the like. In general, see U.S. Patent No. 5,399,346 to Anderson et al.

Co-expression of EN2 and a therapeutic heterologous gene under control of specific DNA recognition sequence by providing a gene therapy expression vector comprising both an EN2 coding gene and a gene under control of, *inter alia*, the EN2 regulatory sequence is described. The most effective use of this mode of treatment is to administer the gene therapy expression vector by known methods to a pregnant woman at risk of carrying an affected fetus. In this context the EN2 regulatory sequence would target the vector to cells types which are affected in autism. Thus if the gene targeting vector is provided during gestation of a fetus, thon the therapeutic gene and/or the EN2 gene itself would be targeted, for example, to neurons which might otherwise fail to develop normally or at all in the fetus. These elements may be provided on separate vectors, *e.g.*, as exemplified *infra*. These elements may be provided in a single expression vector.

### Pharmaceutical Compositions

An EN2 protein or nucleic acid may be a therapeutic compound. Also a therapeutic compound is a drug which increases or decreases, i.e. normalizes (brings to normal levels as described above) the level of EN2 Protein: A drug which normalizes any deleterious downstream effects caused by the presence of SNPS in the BN2 gene are also contemplated. Such drugs may be obtained by assays which detect increase or decrease of EN2 protein or its expression or which normalize the expression of genes which are downstream of the EN2 gene.
A cell-based assay where compounds are tested for the ability to increase EN2 in cultured cells is one such assay. Accordingly a drug which increases the amount of EN2 Protein produced by cultured cells is described.

Pharmaceutical compositions including compounds of this invention, preferably BN2 protein, antibodies, or polynucleotides are also described. Such pharmaceutical compositions may be formulated for administration by any standard means, such as injection, oral, pulmonary, nasal, rectal, etc.

In general, comprehended herein are pharmaceutical compositions comprising effective amounts of a compound, nucleic acid, or protein together with pharmaceutically acceptable diluents, preservatives, solubilizers, emulsifiers, adjuvants and/or carriers. Such compositions include diluents of various buffer content (*e.g.*, Tris-HCl, acetate, phosphate), pH and ionic strength; additives such as detergents and solubilizing agents (*e.g.*, Tween 80, Polysorbate 80), anti-oxidants (*e.g.*, ascorbic acid, sodium metabisulfite), preservatives (*e.g.*, Thimersol, benzyl alcohol) and bulking substances (*e.g.*, lactose, mannitol); incorporation of the material into particulate preparations of polymeric compounds such as polylactic acid, polyglylcolic acid, polyglycolic acid, etc. or into liposomes. Hylauronic acid may also be used.

Such compositions may influence the physical state, stability, rate of *in vivo* release, and rate of *in vivo* clearance of the present proteins and derivatives. *See, e.g.,* Remington's Pharmaceutical Sciences, 18th Ed. (1990, Mack Publishing Co., Easton, PA 18042) pages 1435-1712.

The compositions may be prepared in liquid form, or may be in dried powder, such as lyophilized form. Oral solid dosage forms are described generally in Remington's Pharmaceutical Sciences, 18th Ed.1990 (Mack Publishing Co. Easton PA 18042) at Chapter 89. Solid dosage forms include tablets, capsules, pills, troches or lozenges, cachets or pellets. Formulations for rectal delivery include suppository formulations, which may be similar to those prepared for oral delivery. Also, liposomal or proteinoid encapsulation may be used to formulate the present compositions (as, for example, proteinoid microspheres reported in U.S. Patent No. 4,925,673). Liposomal encapsulation may be used and the liposomes may be derivatized with various polymers (*e.g.*, U.S. Patent No. 5,013,556). A description of possible solid dosage forms for the therapeutic is given by Marshall, K. In: Modern Pharmaceutics Edited by G.S. Banker and C.T. Rhodes Chapter 10, 1979.

In general, the formulation will include the component or components (or chemically modified forms thereof) and inert ingredients which allow for protection against the stomach environment, and release of the biologically active material in the intestine. The active compounds may be chemicaly modified so that oral delivery is efficacious. Generally, the chemical modification contemplated is the attachment of at least one moiety to the component molecule itself, where said moiety permits (a) inhibition of proteolysis; and (b) uptake into the blood stream from the stomach or intestine. Also desired is the increase in overall stability of the component or components and increase in circulation time in the body. Examples of such moieties include: polyethylene glycol, copolymers of ethylene glycol and propylene glycol, carboxymethyl cellulose, dextran, polyvinyl alcohol, polyvinyl pyrrolidone and polyproline.

One may dilute or increase the volume of the therapeutic with an inert material such as carbohydrates, especially mannitol, a-lactose, anhydrous lactose, cellulose, sucrose, modified dextrans and starch. Certain inorganic salts may be also be used as fillers including calcium triphosphate, magnesium carbonate and sodium chloride. Materials used as disintegrates include but are not limited to starch, including sodium starch glycolate, Amberlite, sodium carboxymethylcellulose, ultramylopectin, sodium alginate, gelatin, orange peel, acid carboxymethyl cellulose, natural sponge and bentonite, and insoluble cationic exchange resins. and powdered gums as disintegrants and as binders and these can include powdered gums such as agar, Karaya or tragacanth. Alginic acid and its sodium salt are also useful as disintegrants. Binders may be used to hold the therapeutic agent together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic. An antifrictional agent may be included. Lubricants such as stearic acid including its magnesium and calcium salts, polytetrafluoroethylene (PTFE), liquid paraffin, vegetable oils and waxes,sodium lauryl sulfate, magnesium lauryl sulfate, polyethylene glycol of various molecular weights, Carbowax 4000 and 6000 can be used. Glidants may include starch, talc, pyrogenic silica and hydrated silicoaluminate. A surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethomium chloride. Anionic detergents that could be included are lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. Additives which potentially enhance uptake of the protein are for instance the fatty acids oleic acid, linoleic acid and linolenic acid.

Controlled release oral formulation may be desirable. Pulmonary delivery by known methods, for example as described in U.S. Patent No. 5,451,569, is also contemplated. Vehicles include but are not limited to nebulizers, metered dose inhalers, and powder inhalers. All such devices require the use of suitable formulations. Typically, each formulation is specific to the type of device employed and may involve the use of an appropriate propellant material, in addition to the usual diluents, adjuvants and/or carriers useful in therapy. Also, the use of liposomes, microcapsules or microspheres, inclusion complexes, or other types of carriers is contemplated.

Chemically modified protein may also be prepared in different formulations depending on the type of chemical modification or the type of device employed. Formulations for nasal delivery include those with dextran or cyclodextran. For injectalbe compositions, various standard aqueous formulations are suitable.

### Methods of Treatment

In accordance with the above, the therapeutic compounds can be delivered by standard means including intravenous, intraarterial, intraperitoneal, intramuscular, intracerebral, intraventricular, intrathecal or subcutaneous routes of administration. Alternatively, these compounds, properly formulated, can be administered by nasal, oral, or rectal administration. A constant supply of these therapeutic compounds can be ensured by providing a therapeutically effective dose (*i.e.*, a dose effective to induce normalized (i.e. increased or decreased) levels of EN2 protein in a subject) at the necessary intervals, *e.g.*, daily, every 12 hours, etc. These parameters will depend on the severity of the disease condition being treated, other actions, such as diet modification, that are implemented, the weight, age, and sex of the subject, and other criteria, which can be readily determined according to standard good medical practice by those of skill in the art. Normal levels of EN2 protein may be determined as described above. The subject may be tested to determine whether levels are normalized by, for example, determining the amount of EN2 protein in the cells or tissues of the subject. Similarly dosage may be titrated during treatment by determining whether levels of EN2 protein are increasing or decreasing compared to the subject's levels of EN2 before treatment.

The therapeutic compound can be delivered in a vesicle, in particular a liposome (*see* Langer, Science, 249:1527-1533 (1990); Treat et al., in Liposomes in the Therapy of Infectious Disease and Cancer, Lopez-Berestein and Fidler (eds.), Liss: New York, pp. 353-365 (1989); Lopez-Berestein, ibid, pp. 317-327; see generally *ibid.).* The therapeutic compound can be delivered in a controlled release system, may be administered using intravenous infusion, an implantable osmotic pump, a transdermal patch, liposomes, or other modes of administration. In one example, a pump may be used (*see* Langer, *supra*; Sefton, CRC Crit. Ref. Biomed. Eng., 14:201 (1987); Buchwald et al., Surgery, 88:507 (1980); Saudek et al., N. Engl. J. Med., 321:574 (1989)). In another example, polymeric materials can be used (*see* Medical Applications of Controlled Release, Langer and Wise (eds.), CRC Press: Boca Raton, Florida (1974); Controlled Drug Bioavailability, Drug Product Design and Performance, Smolen and Ball (eds.), Wiley: New York (1984); Ranger and Peppas, J. Macromol. Sci. Rev. Macromol. Chem., 23:61 (1983); see also Levy et al., Science, 228:190 (1985); During et al., Ann. Neurol., 25:351 (1989); Howard et al., J. Neurosurg., 71:105 (1989)). In yet another example, a controlled release system can be placed in proximity of the therapeutic target, *i.e.*, the brain, thus requiring only a fraction of the systemic dose (*see*, *e.g.,* Goodson, in Medical Applications of Controlled Release, supra, vol. 2, pp. 115-138 (1984)).

A subject in whom administration of these therapeutic agents is an effective therapeutic regimen is preferably a human, but can be any animal. The amount administered is an amount effective to normalize the amount of EN2 protein in the subject, whether by increasing or decreasing the amount of protein or the expression of the protein. Thus, as can be readily appreciated by one of ordinary skill in the art, the methods and pharmaceutical compositions described are particularly suited to administration to any animal, *i.e.,* for veterinary medical use.

### Detection of deletions, additions, substitutions and any other mutations

In general the identification of a mutation that involves as little as one nucleotide can be achieved by observing the mutation through nucleotide sequence analysis or a change in an amino acid through amino acid sequence analysis, using techniques known in the art. For example using probes developed from the *EN2* sequence provided herein, or PCR primers provided herein, a suspected mutant gene can be obtained form cells or tissue using known techniques. The gene may then be sequenced by standard methods, and the mutation observed by comparing sequences. Or the gene may be expressed in an expression vector, again using standard protocols, and the resulting protein isolated and sequenced to provide a sequence comparison. An example of a mutant analysis method is single-strand polymorphism mutation screening (SSCP), which employs a PCR-based protocol to accomplish mutation screening. As discussed above, a mutation may comprise one or more deletions, additions, or substitutions of nucleotides. The mutation may be in a coding region, which would affect the sequence of the resulting protein. The mutation may also be in the 5' or 3' noncoding region, which would affect expression of protein, by for example affecting the promoter or enhancer, or by affecting the 3' UTR (affecting RNA stability). A mutation may be as small as one nucleotide, or as large as the entire gene.

### Hybridization Probes

SEQ ID NO: 1, set forth below, is the coding sequence of EN2 DNA. SEQ ID NO: 2, also set forth below, is the complete sequence of the human EN2 protein. The DNA sequences, segments thereof, and sequences complementary in base sequence to said sequences and segments, are preferred hybridization probes in assays for RNA levels. Probes against *EN2* intron sequences, obtainable from the results of the Human Genome Project, can also be used as *EN2* gene-specific probes.

Probes for segments regions of the *EN2* gene or mRNA can be isolated, for example, by cloning the *EN2* gene or cDNA and using PCR to amplify desired regions of the cloned nucleic acid to create a probe population.

Hybridization assays are done under conditions where a probe complementary to a segment of EN2 nucleic acid will preferentially bind to that nucleic acid, as compared to other nucleic acid. An extensive volume of literature exists on various appropriate hybridization conditions for achieving such specificity.

Hybridization can be done by a variety of methods. The optimal solvent composition, probe size, and temperature, will depend on whether the target nucleic acid is for example, in solution, on a solid support (e.g., a nitrocellulose filter) in solution, and or in a cell. They will also depend on whether the probe is in solution or in a solid support (e.g., in a gene chip experiment). The hybridization conditions described for the Northern blot experiments below are illustrative of possible hybridization conditions. Persons in the art will appreciate which conditions are optimal for the various types of possible hybridization experiments.

Single base mutations can, for example, be detected by nucleic acid sequencing, by SSCP, or by hybridization with small probes that require a perfectly complementary target sequence in order to hybridize.

The results of the various assays can be analyzed to determine whether one or both genes have the deletion. For example, if Southern electrophoretic gel analysis of restriction digests of cell DNA reveals the presence of half as much intact EN2 gene DNA as a normal person (as regards the EN2 gene) has, that will indicate that only one of the two gene copies is missing. Also hybridization to chromosomes *in situ* or in chromosomal preparations, using flourescent in situ hybridization or other hybridization techniques, can show if the deletion of a gene is in one or both chromosomes of a chromosome pair. A gel analysis may also reveal an alteration in the pattern of bands from a normal DNA sample. For example a deletion will move its two flanking regions containing restriction sites closer to each other, resulting in different sized fragments than normal DNA when exposed to the same restriction enzyme.

### Demonstration of EN2 defects using protein analysis

In certain examples of the diagnostic methods and kits, protein is isolated from a patient for purposes of determining subnormal levels of protein, especially EN2 protein. Protein may be isolated from cell, tissue, or blood samples using known methods of protein isolation from these materials. In particular, a given protein such as EN2 may be isolated using antibodies. Once isolated, the protein may be sequenced by known methods to determine whether it is a mutant protein. (See, for example, Vladimirov, et al. 1996 Eur. J. Biochem. 239:144-149; Wang and Chait 1997 Methods Mol. Biol. 64:175-182; Hines et al. 1998 J. Protein Chemistry 17:525-526).

### Demonstration of EN2 defects using antibody probes

In particular examples of the diagnostic methods and kits, an antibody probe is used in an assay for purposes of demonstrating the subnormal levels of protein, especially the EN2 protein. Antibodies can be obtained as described above. Also, antibodies that react with EN2 protein have been reported (Ericson, et al. (1992), Science 256:1555-1560; Ericson, et al. (1997), Cell 90:169-180). EN2 size and amount can be determined by Western Blot analysis.

### Autism

Autism includes any form of autism. Such diseases are currently denoted as autism, an autistic spectrum disorder which includes Asperger's Syndrome and Pervasive Developmental Disorder (PDD-NOS).

The "DSM-IV" criteria for autistic disorder are those set forth in the American Psychiatric Association's Diagnostic and Statistical Manual of Mental Disorders, 4th edition, Washington, DC, 199 pp70-71. The diagnosis is based on the presence of 6 or more diagnostic criteria from three possible Groups 1, 2, and 3 with at least two of the criteria being from Group 1, at least one from Group 2, and at least one from Group 3. The three groups correspond to three core symptoms:
Group 1 - Qualitative impairment in social interaction.
Group 2 - Qualitative impairment in communication.
Group 3 - Restricted repetitive and stereotyped patterns of behavior, interests and activities.

The four diagnostic criteria in Group 1 are: (i) marked impairment in multiple nonverbal behaviors (e.g., eye to eye gaze, facial expression, body postures, and gestures to regulate social interaction); (ii) failure to develop peer relationships appropriate to developmental level; (iii) absence of spontaneous seeking to share enjoyment, interests, or achievements with others (e.g., lack of showing, bringing, or pointing out objects of interest); and (iv) absence of social or emotional reciprocity.

The four diagnostic criteria in Group 2 are (i) delay in, or total absence of spoken language development (without an attempt to compensate through alternative modes of communication, such as gesture or mime); (ii) adequate speech but marked impairment in ability to initiate or sustain a conversation with others; (c) stereotyped and repetitive use of language or idiosyncratic language; and (iv) absence of varied, spontaneous make-believe play or social imitative play appropriate to developmental level.

The four diagnostic criteria in Group 3 are: (i) encompassing preoccupation with one or more stereotyped and restricted patterns of interest that is abnormal either in intensity or focus; (ii) inflexible adherence to specific nonfunctional routines or rituals, stereotyped and repetitive motor mannerisms (e.g., hand or finger flapping or twisting, or complex whole-body movements); and (iv) persistent preoccupation with parts of objects.

Diagnosis of autism using the DSM-IV criteria, followed by confirmation of the diagnosis by the Autism Diagnostic Interview-Revised (ADI-R), provides good results. The ADI-R is a well known review process for establishing autism (see for example Lord et aL, 1994 J. Autism Dev Disord. 2:659-85; and Le Couteur, et al. 1989 J Autism Dev Disord. 19:363-87).

In the Examples described here, the clinical diagnosis was determined by the DSM-IV criteria an then each individual was then subjected to the Autism Diagnostic Interview-Revised (ADI-R). The autistic individuals were identified by having at least six of the items described above.

### Modulation of Downstream Genes

Genes that are directly regulated by EN2 are also described Mutations in EN2 may result in increase or decrease of the expression of these genes. Such downstream genes may be expressed at increased or decreased levels when EN2 is downregulated, and this is one means of isolating such genes using known methods. For example cerebellar anlage (e10.5) could be dissected from EN2-/- knockout mice and wild-type littermates and their mRNA isolated to determine which are expressed at lower levels. For example, the mRNA could be hybridized against microarrays containing all the genes from the mouse genome. Genes directly regulated by EN2 such as PCP2 (L7) (Sanlioglu et al (1998) J Neurobiol. 36:559-71) may be expressed at higher or lower levels than the others.

### EXAMPLES

The following examples are put forth so as to provide those of ordinary skill in the art with a complete disclosure and description of how to practice the invention and are not intended to limit the scope of what the inventors regard as their invention.

### Example 1

Families recruited to the Autism Genetic Resource Exchange (AGRE) were used for these studies. AGRE is a central repository of family DNA samples created by The Cure Autism Now (CAN) Foundation and the Human Biological Data Interchange. The selection criteria require that at least two family members have a diagnosis of autism, Asperger's Syndrome or PDD-NOS. The diagnosis and characteristics of these families are described in detail elsewhere (Geschwind et al (2001), Am J Hum Genet. 69:463-6; Liu et al (2001), Annu Rev Neurosci 24:869-896). For our analysis, a narrow diagnosis is defined as only autism while a broad diagnostic includes individuals affected with either autism, Asperger's Syndrome or PDD-NOS. In this study, 137 triads (parent-offspring trios) under the narrow diagnostic were initially evaluated. This was then extended to a total of 166 pedigrees that include 310 triads and 168 phenotypic discordant sib pairs (DSP) under the broad diagnosis (740 total subjects). For the narrow diagnostic, 163 pedigrees with 250 triads and 134 DSP were analyzed (n = 672).

*EN2* spans 8kb of the genome and is encoded by two exons separated by a single intron. SNPs within the *EN2* gene were initially identified through genome browsers. Two SNPs (*rs1861972* and *rs1861973*) located 152 bp apart in the intron were initially verified by DNA sequence analysis of 24 individuals.

**Table 1. TDT results for rs1861972 and rs1861973 SNPs in triad families.**

| DbSNP No | Polymorphism^{a} | Frequency^{b} | T^{c} | U^{c} | *P*-value^{d} | GRR^{e} | PAR^{f} |
|---|---|---|---|---|---|---|---|
| rs1867972 | A/G | 0.297 | 77 | 41 | 0.0009 | 1.88 | 62% |
| rs1861973 | C/T | 0.292 | 70 | 35 | 0.0006 | 2.00 | 66% |

| | | | | | | | |
|---|---|---|---|---|---|---|---|
| ^{a}-Second allele is rare allele. ^{b}-Frequency of rare allele. ^{c}-T=number of times frequent allele transmitted and U=number of times frequent allele not transmitted from heterozygous parents. ^{d}-Two-sided P-value (1df) corresponding to χ² generated by TRANSMIT. ^{e}-Genotype relative risk estimated by transmission ratio (T/U) assuming a multiplicative model. ^{f}-Population attributable risk calculated as PAR=(X-1)/X; where X=(1-f)² + 2f(1-f)γ + f²γ² and γ=GRR. | | | | | | | |

The tetra-primer ARMS-PCR strategy was then used to genotype family members (Ye et al. (2001), Nucleic Acids REN29:E88-8). Primers were selected using a web based software program and PCR conditions were determined that allowed the specific amplification of all possible genotypes. Genotypes for both SNPs were determined for the initial 137 triads that displayed just the narrow diagnostic and were verified to be in Hardy Weinberg equilibrium. The frequencies for the A and G alleles of *rs1861972* are 70.3 and 29.7 respectively (Table 1). Similar values are observed for *rs1861973* (C=70.8 and T=29.2) (Table 1). There is significant positive linkage disequilibrium (LD) between the alleles of these markers (delta = 0.82, delta-χ²= 227.96, df = 1, P <0.00001) (Hill and Weir (1994), Am. J. Hum. Genet., 54: 705-714).

Allelic transmission distortions were assessed for each SNP by the transmission disequilibrium test (TDT) (Spielman et al. (1993), Am J Hum Genet 52: 506-516), using the TRANSMIT program (Clayton (1999), Am J Hum Genet 65: 1170-1177). Excess transmission of the *rs1861972* A-allele to affected offspring was observed, with 77 of 118 heterozygous parents (65%) transmitting the A-allele and only 41 transmitting the G allele (McNemar's χ²=10.98, df=1, P=0.0009) (Table 1). Under a multiplicative model, the transmission ratio Transmitted/Untransmitted (T/U) is an estimator of genotype relative risk (GRR) (Altshuler et al. (2000), Nature Genet 26: 76-80). The GRR attributable to the A allele of the *rs1861972* SNP is estimated to be 1.88 which in turn corresponds to a population attributable risk (PAR) (Altshuler et al. (2000), Nature Genet 26: 76-80) of approximately 62%. In other words these data indicate that if the population were monomorphic for the G (protective) allele the prevalence of autism would be 62% lower. Since the GRR and PAR are calculated from a sample population ascertained for multiple affected siblings, the actual risk estimates may be different in the general AS population.

A similar distortion of transmissions was observed for the *rs1861973* SNP with 70 of 105 heterozygous parents (67%) preferentially transmitting the C allele to their Autistic offspring (McNemar's χ²=11.67, df=1, P=0.0006) (Table 1). The C allele of the *rs1861973* SNP provides a GRR of 2.00 with a corresponding PAR of 66%.

Haplotype transmission/disequilibrium tests were then performed again using TRANSMIT software. In our population, the A,C haplotype has an observed frequency of 67.6%. This haplotype is specifically overtransmitted (80 transmitted vs. 36 untransmitted) to affected individuals (χ²=16.07, df=1, *P*=.000062) (Table 2). The G,T, A,T and G,C haplotypes are all undertransmitted (Table 2). The global χ² test based upon the common haplotypes A,C and G,T (frequency > 5%) was then calculated to be χ²=18.90 (df=2), with a corresponding P value of 0.000079 (Table 2). These tests were then reiterated 50000 times by a bootstrap simulation procedure to control for ambiguous haplotypes. Similar empirical P-values as before were obtained (*P*=0.00012)(Table 2). The GRR attributable to the A,C haplotype was found to be 2.19 with a corresponding PAR of 69%. Together this analysis demonstrates that the *EN2* A,C haplotype is overtransmitted and is a high risk haplotype in autistic individuals.

**Table 2. Haplotype TDT analysis in triads.**

| Haplotype | Frequency | T | U | *P*-value^{a} |
|---|---|---|---|---|
| A, C^{b} | 0.676 | 79 | 36 | 0.000062 |
| A, T | 0.032 | 4 | 12 | 0.0455 |
| G, C | 0.028 | 3 | 10 | 0.0525 |
| G, T | 0.262 | 39 | 67 | 0.0065 |
| Global^{c} | | | | 0.000079 |
| Global^{d} | | | | 0.00012 |

| | | | | |
|---|---|---|---|---|
| ^{a} -Two-sided P-value corresponding to χ² generated by TRANSMIT. ^{b}-GRR=2.19, PAR=69%. ^{c}-Global χ² test based on combined common haplotypes, frequency greater than 5% (2df). ^{d}-Bootstrap simulation of global χ² test (50000 reiterations). | | | | |

To determine whether these findings could be extended to a larger sample, affected and unaffected siblings from the original 137 triads and 29 additional families were examined. The inheritance of each SNP was then assessed in these extended pedigrees using the Pedigree Disequilibrium Test (PDT)(Martin et al. (2000), Am J Hum Genet 67: 146-154). This program was used in place of TRANSMIT for the analysis of the extended pedigrees since the inevitable non-independence of affected siblings would render analysis conducted using TRANSMIT and other similar programs as invalid tests of association though they remain appropriate for linkage analysis. PDT has been specifically designed to overcome this limitation by allowing the use of data from related triads and discordant sibships from extended families when testing for LD.

For *rs1861972*, the A allele is again overtransmitted from heterozygous parents when individuals under a broad or narrow diagnostic are included in the analysis (narrow: 120 transmitted vs. 82 untransmitted; and broad: 150 transmitted vs. 107 untransmitted). The A allele is also over-represented in affected sibs of DSPs (defined by both genotypic and phenotypic discordance) under both diagnostics (narrow: 67 alleles in affected and 50 in unaffected sibs of 44 genotypic DSPs; broad: 89 in affected and 63 in unaffected of 57 genotypic DSPs). These results are significant when analyzed by PDT (narrow *P*=0.0106, broad *P*=0.0050)(Table 3).

**Table 3. PDT analysis of rs1867972 and rs1867973 in the extended pedigrees.**

| | | Parental | | | | | |
|---|---|---|---|---|---|---|---|
| | | Transmissions | | DSP^{a} | | | |
| dbSNP No. | Diagnostic | T | U | A | UA | Total | P-value^{b} |
| rs1867972 | narrow | 120 | 82 | 67 | 50 | 44 | 0.0106 |
| rs1867972 | broad | 150 | 107 | 89 | 63 | 57 | 0.0050 |
| rs1867973 | narrow | 108 | 68 | 59 | 40 | 37 | 0.0033 |
| rs1867973 | broad | 129 | 90 | 73 | 50 | 46 | 0.0063 |

| | | | | | | | |
|---|---|---|---|---|---|---|---|
| ^{a}-Discordant sibpair counts: A= total number of the common allele in affected siblings and UA=total number of the common allele in unaffected siblings of genotypic discordant DSPs; Total=total number of genotypic discordant DSPs. ^{b}-*P*-value generated by PDT. | | | | | | | |

Analysis for the *rs1861973* SNP revealed a similar pattern. The C allele is again overtransmitted to affected individuals (narrow: 108 transmitted vs. 68 untransmitted, broad: 129 transmitted vs. 90 untransmitted) and over-represented in affected siblings of DSPs (narrow: 59 in affected and 40 in unaffected of 37 genotypic DSPs; broad: 73 vs. 50 in affected and unaffected sibs respectively of 46 genotypic DSPs). This inheritance pattern is significant under the broad and narrow diagnosis when analyzed by PDT (narrow *P*=0.0033, broad *P*=0.0063)(Table 3). Thus, *rs1861972* and *rs1861973* demonstrate significant association to both autism and related AS disorders.

Haplotype analysis on the extended pedigrees was then performed. Since the two SNPs are in tight LD and are only 152 bp apart from each other, it is extremely unlikely for a recombination event to occur between them in a sample of this size. In the absence of recombination, unambiguous haplotypes could be assigned to all individuals in 160 of the 166 pedigrees. In only 6 families the parental haplotype phase could not be distinguished. These pedigrees were omitted for further analysis. Each haplotype was recoded as a single allele. By recoding the haplotypes as a single locus, the transmissions could be analyzed by PDT. The A,C haplotype is again overtransmitted under both diagnostic classifications (narrow: 120 transmitted vs. 66 untransmitted, broad: 143 transmitted vs. 86 untransmitted) and over-represented in affected siblings of DSPs (narrow: 64 vs. 45 from 44 genotypic DSPs; broad: 76 vs. 53 from 52 genotypic DSPs) at significant levels (narrow *P*=0.0007, broad *P*=0.0009)(Table 4). Conversely, the A,T, G,C and G,T haplotypes are all undertransmitted. Global χ² tests for all haplotypes also yielded significant *P*-values (narrow *P*=0.0003, broad *P*=0.0005)(Table 4). These results demonstrate significant association between *EN2* and autism spectrum disorders.

**Table 4. Extended pedigree haplotype analysis.**

| | | Parental | | | | | |
|---|---|---|---|---|---|---|---|
| | | Transmissions | | DSP | | | |
| Haplotype^{a} | Diagnostic | T | U | A | UA | Total | *P*-value^{b} |
| A,C | narrow | 120 | 66 | 64 | 45 | 44 | 0.0007 |
| A,T | narrow | 7 | 21 | 1 | 3 | 4 | 0.0136 |
| G,C | narrow | 7 | 20 | 3 | 5 | 8 | 0.0997 |
| G,T | narrow | 69 | 96 | 17 | 30 | 35 | 0.0299 |
| Global | narrow | | | | | | 0.0003 |
| A,C | broad | 143 | 86 | 76 | 53 | 52 | 0.0009 |
| A,T | broad | 10 | 25 | 1 | 3 | 4 | 0.0195 |
| G,C | broad | 9 | 26 | 4 | 5 | 9 | 0.0587 |
| G,T | broad | 92 | 117 | 20 | 40 | 43 | 0.0634 |
| Global | broad | | | | | | 0.0005 |

| | | | | | | | |
|---|---|---|---|---|---|---|---|
| ^{a}-Haplotypes recoded as alleles prior to PDT analysis. ^{b}-P-value generated by PDT. | | | | | | | |

The haplotype transmissions were also investigated for parent of origin and sex differences using ETDT (Sham and Curtis (1995), Ann Hum Genet 59: 323-336) and TRANSMIT software respectively. When maternal and paternal transmissions were investigated separately, no parental differences in transmission ratios was observed (data not included). Male and female affected individuals are also equally likely to inherit the A,C haplotype (data not included). Thus, these data do not support a significant parent of origin or sex bias effects.

Computer prediction programs indicate that these polymorphisms do not introduce new splice acceptor or donor sites. Similar analysis has determined that the A and G alleles of *rs1861972* do not alter any putative transcription factor binding sites in the intron. However, the C allele for *rs1861973* generates a consensus binding site for the general transcription factor, Sp1. This potential difference in transcription factor binding might alter the transcriptional regulation of *EN2* and confer risk to autism.

### Results

These data represent the most significant genetic association reported for AS disorders. The overtransmission of the A and C alleles of both SNPs indicate that either they are risk causing or these SNPs are in linkage disequilibrium with other polymorphisms that contribute to autism.

The results of our association study together with the similarities in the cerebellar phenotype observed in the mouse knockout and autistic individuals make *EN2* an excellent candidate for an autism susceptibility locus. Using the transmission data observed in our sample we have estimated a genotype relative risk of 2.19 and a population attributable risk of 69% for the A,C predisposing haplotype. In other words these data indicate that if the population were monomorphic for the G and T (protective) alleles the prevalence of autism would be 69% lower. These data suggest a highly significant role for variation at this locus in the etiology of AS. Since the GRR and PAR are calculated from a sample population ascertained for multiple affected siblings the actual risk estimates may be different in the general AS population. Estimates of GRR in multiplex sibships compared with singleton families are thought to be distorted depending on the background heritability (multilocus model of the disorder). The GRR is in fact expected to be deflated at a rate that is proportional to an increase in background heritability and more accurately estimated in situations of low background heritability (Risch N (2001) Theor Popul Biol 60:215-220). Given the complex polygenic model of inheritance with anywhere between 3-15 interacting loci that has been proposed for AS, it is not clear what if any the effect of the true model in our sample has been on our risk estimates. Further studies in other population or single-ascertainment family based datasets should confirm the true impact of this locus.

*En2* functions in cerebellar patterning during development. Although *En2* is expressed as early as embryonic day 9.5 during mouse cerebellar development, it is believed to be redundant to *En1* in anterior-posterior patterning of the neural tube necessary to form the cerebellum (Hanks et al (1995), Science 269:679-682; Liu and Joyner (2001), Annu Rev Neurosci 24:869-896). The knockout has demonstrated that *En2* is essential for normal postnatal cerebellar development (Millen et al (1994), Development 120:695-706; Millen et al (1995), Development 121:3935-45; Kuemerle et al (1997), J Neurosci 17:7881-9). Although the cerebellum is often noted for its uniform structure, anatomical and biochemical studies have determined that the cerebellum is highly patterned. A number of genes including *En2* are expressed in stripes in the postnatal cerebellum. These genes are believed to coordinate both the differential proliferation that is potentially responsible for proper cerebellar foliation as well as the topographic mapping of spinocerebellar and olivocerebellar afferents (Hawkes and Eisenmann (1997), Perspect Dev Neurobiol 5:95-105). In the *En2* knockout, this postnatal patterning is disrupted as evidenced by abnormal gene expression, foliation and misrouting of afferents (Vogel et al (1996), Brain Res Dev Brain Res 96:210-218; Kuemerle et al (1997), J Neurosci 17:7881-9). This abnormal patterning could either directly affect cerebellar function or indirectly perturb interacting forebrain structures that mediate altered social, language and cognitive abilities observed in autism. Future studies will determine whether polymorphisms in human *EN2* affect the same developmental processes.

### Example 2

To investigate whether the findings of Example 1 could be replicated in additional populations, 239 further families from AGRE and an additional 141 autistic families from a separate collection funded by the National Institute of Mental Health (NIMH) and ascertained by the group at Stanford University (Risch et al (1999) Am J Hum Genetics 65:493-507) were analyzed. Table 5 lists the number of families, triads and DSPs analyzed for each population. These additional samples were genotyped for *rs1861972* and *rs1861973* and evidence for association with ASD was examined in these populations.

Transmission disequilibrium analysis in the original study of 167 AGRE families revealed that the A allele of *rs1861972* and the C allele of *rs1861973* were both overtransmitted from heterozygous parents and were over-represented in affected siblings compared to unaffected siblings of DSPs (Gharani et al (2004) Mol Psychiatry 9:474-484). Association of *rs1861972* and *rs1861973* was now tested in the newly acquired datasets (Table 5) by using PDTPHASE (version 2.404) program (Dudbridge (2003) Genetic Epidim. 25:115-121), which is a modification of the pedigree-based transmission disequilibrium test PDT (Martin et al (2000) Am J Hum Genetics 67:146-154). PDTPHASE has a number of advantages over the PDT program. First, it can handle missing parental data. PDT only includes triads from families for which genotype data is available from both parents. There are 63 out of 547 families with one parent missing in the total set of families (AGRE and NIMH). These families were excluded from analysis by PDT resulting in a loss of power. Second, PDTPHASE is able to perform multilocus analysis. Furthermore, PDTPHASE includes an EM algorithm that calculates maximum-likelihood gametic frequencies under the null hypothesis allowing the inclusion of phased uncertain haplotypes. In Example 1, haplotype analysis was conducted using the PDT program. Since PDT is designed for single locus analysis, each unambiguous haplotype (constructed using SIMWALK version 2.86) was recoded as a single allele to allow haplotype transmission testing. Thus, all families were utilized in the analysis including those that would be excluded by the previous method (based on missing parental data and ambiguous haplotypes) again increasing the statistical power of the analysis.

**Table 5 Number of Families, Triads and Discordant Sib Pairs (DSPs) analyzed**

| Population | Diagnosis | Families^{c} | Triads^{c} | DSPs^{c} |
|---|---|---|---|---|
| | | | | |
| AGRE^{a} | narrow | 227 | 371 | 270 |
| | broad | 238 | 467 | 320 |
| | | | | |
| NIMH | narrow | 142 | 253 | 75 |
| | broad | 144 | 264 | 77 |
| | | | | |
| AGRE | narrow | 391 | 633 | 408 |
| TOTAL^{b} | broad | 404 | 792 | 492 |
| | | | | |
| NIMH + AGRE | narrow | 533 | 886 | 483 |
| TOTAL^{c} | broad | 548 | 1056 | 569 |

| | | | | |
|---|---|---|---|---|
| ^{a}- Population is comprised of 239 additional AGRE families ^{b}- The 167 initial AGRE families and the recently obtained 239 AGRE families are referred to as AGRE TOTAL ^{c}- Number of families, triads, and DSPs used by the PDTPHASE program. | | | | |

Results of the single marker analyses are presented in Table 6. Analysis of *rs1861972* in the newly acquired AGRE families failed to provide significant association under the narrow diagnosis (*P*=0.1229) (Table 6). When additional family members under the broad diagnosis were added to the analysis, nominally significant association of *rs1861972* with ASD was observed (*P*=0.0401)(Table 6). For *rs1861973*, similar results were obtained. Under the narrow diagnosis, significant association was not observed (*P*=0.0575) (Table 6). However, like *rs1861972*, when additional individuals diagnosed under broad criteria were added to the analysis, significant association was obtained (*P*=0.02)(Table 6).

**Table 6. rs1861972 and rs1861973 replication results in extended pedigrees.**

| | | Parental Transmissions ^{c} | | | DSP ^{d} | | | |
|---|---|---|---|---|---|---|---|---|
| Population | SNP | Diagnosis | T | U | A | UA | χ^{2 c} | *P*-value ^{f} |
| AGRE^{a} | *rs1861972* | narrow | 479 | 451 | 387 | 374 | 2.175 | 0.1403 |
| | | broad | 599 | 559 | 463 | 443 | 3.939 | 0.0471 |
| | *rs1861973* | narrow | 474 | 438 | 381 | 362 | 3.805 | 0.0511 |
| | | broad | 590 | 546 | 457 | 430 | 5.357 | 0.0206 |
| | | | | | | | | |
| NIMH | *rs1861972* | narrow | 240 | 224 | 119 | 113 | 2.327 | 0.1272 |
| | | broad | 246 | 230 | 121 | 115 | 2.241 | 0.1344 |
| | *rs1861973* | narrow | 245 | 227 | 117 | 110 | 3.141 | 0.0764 |
| | | broad | 252 | 233 | 119 | 111 | 3.522 | 0.0606 |
| | | | | | | | | |
| AGRE TOTAL ^{b} | *rs1861972* | narrow | 860 | 806 | 597 | 567 | 5.929 | 0.0149 |
| | | broad | 1069 | 1001 | 724 | 677 | 8.93 | 0.0028 |
| | *rs1861973* | narrow | 845 | 777 | 588 | 553 | 9.091 | 0.0026 |
| | | broad | 1042 | 967 | 707 | 658 | 10.71 | 0.0011 |
| | | | | | | | | |
| NIMH+AGRE | *rs1861972* | narrow | 1100 | 1030 | 716 | 680 | 8.037 | 0.0046 |
| TOTAL | | broad | 1315 | 1231 | 845 | 792 | 11.06 | 0.0009 |
| | *rs1861973* | narrow | 1090 | 1004 | 705 | 663 | 11.994 | 0.0005 |
| | | broad | 1294 | 1200 | 826 | 769 | 13.878 | 0.0002 |

| | | | | | | | | |
|---|---|---|---|---|---|---|---|---|
| ^{a}- Population is comprised of 239 additional AGRE families. ^{b}- The 167 initial AGRE families and the recently obtained 239 AGRE families are referred to as AGRE TOTAL. ^{c}- T=number of times frequent allele transmitted; U=number of times frequent allele not transmitted from parents. ^{d}- Discordant sibpair counts: A=total number of the common allele in affected siblings; UA=total number of the common allele in unaffected siblings of DSPs. ^{c}- Global χ² values calculated by PDTPHASE^{sum} ^{f}- *P*-value generated by PDTPHASE^{sum} (1df). | | | | | | | | |

The same pedigree disequilibrium test was then performed on the NIMH dataset (Table 6). No significant evidence for association was obtained for either intronic SNP under both the narrow and the broad diagnosis (*rs1861972*-narrow: *P*=0.1272, broad: *P*=0.1344; *rs1861973*-narrow: *P*= 0.0764, broad: *P*= 0.0606)(Table 6).

Since identical criteria was used to obtain all the AGRE pedigrees, the previously tested 167 AGRE dataset was combined with the recently acquired AGRE families and evidence for association of the intronic markers was tested once again. By combining these datasets more significant association was observed for *rs1861972* and *rs1861973* under both the narrow and the broad diagnosis than in either dataset individually (*rs1861972*-narrow: *P*=0.0130, broad: *P*= 0.0028; *rs1861973*-narrow: *P*=0.0025, broad: *P*= 0.0009) (Table 6). Next, to investigate whether the NIMH dataset might also add to the transmission disequilibrium, the pedigrees from all three populations were combined and analyzed by PDTPHASE. Again more significant association was observed under both diagnostic criteria than in any population individually or in the entire AGRE dataset (*rs1861972*-narrow: *P*= 0.0046, broad: *P*=0.0009; *rs1861973*-narrow: *P*= 0.0005, broad: *P*=0.0002) (Table 6). Thus, evidence for association of *rs1861972* and *rs1861973* with ASD is observed in all three populations.

As set forth in Example 1, *rs1861972* and *rs1861973* display significant LD with each other. This significant LD relationship is also observed in both the additional 239 AGRE (D'=0.967) and 141 NIMH families (D'=0.977). The allele frequencies for *rs1861972* and *rs1861973* in the two new populations are also almost identical to each other and to what was reported previously (239 AGRE: *rs1861972* G allele-0.29, *rs1861973* C allele-0.31; 141 NIMH: *rs1861972* G allele -0.29, *rs1861973* C allele-0.30).

The similarity in the inter-marker LD strength and the SNP allele frequencies between the three data sets indicates that they are likely to be derived from the same population and are therefore likely to share a similar LD relationship with the putative etiological variant. To investigate this further, *rs1861972-rs1861973* haplotype transmissions were examined in each of the populations under both the broad and the narrow diagnosis using PDTPHASE. In the recently acquired 239 AGRE pedigrees, significant excess transmission of the *rs1861972-rs1861973* A-C haplotype to affected offspring was observed under both narrow and broad diagnostic criteria (narrow: *P*=0.033; broad: *P*=0.011) (Table 7). The A-T, G-C and G-T haplotypes were all under-transmitted (Table 7) and global χ2 tests for all haplotypes yielded significant *P*-values (narrow: P=0.0093; broad: *P*=0.0028).

**Table 7. rs1861972-rs1861973 haplotype replication results in extended pedigrees.**

| | | Parental Transmissions ^{c} | | | DSP ^{d} | | | |
|---|---|---|---|---|---|---|---|---|
| Population | Haplotype | Diagnosis | T | U | A | UA | χ^{2e} | *P*-value ^{f} |
| AGRE^{a} | A-C | narrow | 470 | 429 | 381 | 362 | | 0.03367 |
| | A-T | | 4 | 16 | 2 | 5 | | - |
| | G-C | | 2 | 8 | 0 | 0 | | - |
| | G-T | | 176 | 199 | 145 | 161 | | 0.1552 |
| | global | | | | | | 9.339 | 0.00938 |
| | | | | | | | | |
| | A-C | broad | 584 | 533 | 457 | 430 | | 0.01104 |
| | A-T | | 6 | 18 | 2 | 6 | | - |
| | G-C | | 2 | 11 | 0 | 0 | | - |
| | G-T | | 226 | 256 | 167 | 190 | | 0.07547 |
| | global | | | | | | 11.69 | 0.0029 |
| | | | | | | | | |
| NIMH | A-C | narrow | 239 | 222 | 117 | 110 | | 0.09611 |
| | A-T | | 1 | 2 | 0 | 1 | | - |
| | G-C | | 6 | 5 | 0 | 0 | | - |
| | G-T | | 88 | 105 | 29 | 35 | | 0.2891 |
| | global | | | | | | 3.688 | 0.1582 |
| | | | | | | | | |
| | A-C | broad | 245 | 228 | 119 | 111 | | 0.09118 |
| | A-T | | 1 | 2 | 0 | 2 | | - |
| | G-C | | 7 | 5 | 0 | 0 | | - |
| | G-T | | 91 | 109 | 31 | 37 | | 0.09126 |
| | global | | | | | | 3.839 | 0.1467 |
| | | | | | | | | |
| AGRE TOTAL ^{b} | A-C | narrow | 837 | 755 | 584 | 549 | | 0.00077 |
| | A-T | | 12 | 39 | 7 | 9 | | - |
| | G-C | | 6 | 21 | 4 | 4 | | - |
| | G-T | | 285 | 325 | 197 | 230 | | 0.02966 |
| | global | | | | | | 20.19 | 0.0000412 |
| | | | | | | | | |
| | A-C | broad | 1031 | 940 | 703 | 654 | | 0.00027 |
| | A-T | | 17 | 44 | 7 | 10 | | - |
| | G-C | | 7 | 25 | 4 | 4 | | - |
| | G-T | | 365 | 411 | 236 | 282 | | 0.0138 |
| | global | | | | | | 22.05 | 0.0000163 |
| | | | | | | | | |
| NIMH+ | A-C | narrow | 1076 | 977 | 701 | 659 | | 0.00018 |
| AGRE | A-T | | 13 | 41 | 7 | 10 | | - |
| TOTAL | G-C | | 12 | 26 | 4 | 4 | | - |
| | G-T | | 373 | 430 | 226 | 265 | | 0.00833 |
| | global | | | | | | 23.05 | 0.00000985 |
| | | | | | | | | |
| | A-C | broad | 1276 | 1168 | 822 | 765 | | 0.0000607 |
| | A-T | | 18 | 46 | 7 | 12 | | - |
| | G-C | | 14 | 30 | 4 | 4 | | - |
| | G-T | | 456 | 520 | 267 | 319 | | 0.00371 |
| | global | | | | | | 24.89 | 0.00000392 |

| | | | | | | | | |
|---|---|---|---|---|---|---|---|---|
| ^{a}- Population is comprised of 239 additional AGRE families. ^{b-} The 167 initial AGRE families and the recently obtained 239 AGRE families are referred to as AGRE TOTAL. ^{c}- T=number of times frequent allele transmitted; U=number of times frequent allele not transmitted from parents. ^{d}- Discordant sibpair counts: A=total number of the common allele in affected siblings; UA=total number of the common allele in unaffected siblings of DSPs. ^{e}- Global χ² values calculated by PDTPHASE^{sum}, restricted to common haplotypes with frequency greater than 5%. ^{f}- *P*-value generated by PDTPHASE^{sum} (1df for single haplotype and 3df for the global tests). | | | | | | | | |

Haplotype transmissions were then examined for the NIMH dataset and no evidence for over-transmission was observed under both the narrow and the broad diagnoses for any of the A-C, A-T, G-C, and G-T haplotypes (Table 7). Global χ2 test also yielded non-significant *P*-values under both the narrow and the broad diagnostic criteria (narrow: *P* = 0.1582; broad: *P* = 0.1467)(Table 7).

Next, the original 167 AGRE pedigrees were combined with the recently acquired 239 AGRE families and the haplotype transmissions were analyzed for the total AGRE samples. As for the individual SNP analysis, greater significance of association for the A-C haplotype was now observed in the combined dataset than in either population individually (narrow: *P*=0.0007; broad: *P*= 0.00026) (Table 7). The A-T, G-C, and G-T haplotypes were all under-transmitted to affected individuals and global χ2 test for all haplotypes yielded very significant *P*-values under both the narrow and the broad diagnosis (narrow: *P*=0.0000412; broad: *P*=0.00000162) (Table 7). Finally, when all 3 populations were combined, the most significant *P* values were observed for both the A-C haplotype (narrow: *P*=0.00018; broad: *P*=0.00000607)(Table 7) and the global χ2 (narrow: *P*=0.00000985; broad: *P*=0.00000392). Consistent with the single marker analysis, evidence for association of the *rs1861972-rs1861973* haplotype with ASD is observed in all three populations.

Given the increased power of this larger data set, *rs1861972-rs1861973* haplotype transmissions were re-examined for sex differences and parent-of-origin effects. Previously, 138 triads from the 167 AGRE families were analyzed and no evidence for either a sex bias or a parent of origin effect was observed. However, given the small sample size, these stratified analyses were likely to be underpowered. In the 547 families comprising the total dataset, there are 191 and 694 affected females and males respectively under the narrow diagnosis and 237 affected females and 816 affected males under the broad analysis. When the *rs1861972-rs1861973* haplotype transmissions were analyzed by sex of offspring, the *P*-values obtained for transmissions to male offspring (narrow: *P*=0.00040; broad: *P*=0.00092) were more significant than those obtained for the transmissions to female offspring (narrow: *P*=0.049; broad: *P*=0.021)(Table 8A). To assess whether this is due to the greater number of affected males than females in the data set, 2 by 2 contingency tables and the Fisher's exact test were used to compare the transmissions of the A-C *rs1861972-rs1861973* haplotype to male and female offspring under the narrow and broad diagnosis. No significant difference between the transmission ratios was observed under both diagnostic schemes (narrow: Fisher's exact one-sided *P*=0.455; broad: Fisher's exact one-sided *P*=0.340), indicating that male and female affected individuals are equally likely to inherit the A-C *rs1861972-rs1861973* haplotype.

**Table 8A: rs1861972-rs1861973 sex bias haplotype analysis**

| Population | Transmissions to: | | narrow | | | broad | | |
|---|---|---|---|---|---|---|---|---|
| | | | T^{b} | UT^{b} | *P*-value^{c} | T^{b} | UT^{b} | *P*-value^{c} |
| AGRE | male offspring | A-C | 826 | 753 | 0.0004 | 971 | 898 | 0.0009 |
| TOTAL^{a} | | A-T | 10 | 32 | 0.0019 | 11 | 36 | 0.0005 |
| & NIMH | | G-C | 11 | 23 | 0.05 | 13 | 26 | 0.063 |
| | | G-T | 295 | 334 | 0.024 | 357 | 392 | 0.049 |
| | | global | | | 0.000036 | | | 0.000047 |
| | | | | | | | | |
| | female offspring | A-C | 250 | 224 | 0.049 | 305 | 270 | 0.02 |
| | | A-T | 3 | 9 | 0.18 | 7 | 10 | 0.52 |
| | | G-C | 1 | 3 | 0.16 | 1 | 4 | 0.08 |
| | | G-T | 78 | 96 | 0.13 | 99 | 128 | 0.045 |
| | | global | | | 0.0585 | | | 0.0227 |

| | | | | | | | | |
|---|---|---|---|---|---|---|---|---|
| ^{a}- The 167 initial AGRE families and the recently obtained 239 AGRE families are referred to as AGRE TOTAL. ^{b}- T=number of times frequent allele transmitted; U=number of times frequent allele not transmitted from parents. ^{c}- *P*-value generated by PDTPHASE (1df for single haplotype and 3df for the global tests). | | | | | | | | |

To investigate possible parent-of-origin effects, maternal and paternal transmissions for the entire dataset were analyzed separately using TDTPHASE (version 2.404) and included transmissions from heterozygous parents to a single affected offspring per family. The paternal and maternal transmission data were then compared with each other and with the combined parental transmissions under both diagnostic schemes (Table 8B). Under the narrow diagnosis, there were 336 A-C/- heterozygous parents of which 172 were heterozygous fathers and 164 were heterozygous mothers. Analysis of the combined parental data demonstrated that 204 of 336 heterozygous parents (60.7%) transmitted the A-C haplotype and the overall haplotype transmission distortion was found to be highly significant (*P*=0.000072). Analysis by sex of parent revealed that both parents significantly overtransmitted the A-C haplotype to affected offspring (paternal transmissions *P*=0.021, maternal transmissions *P*=0.00025)(Table 8B). The maternal transmissions demonstrated a greater distortion of the transmission ratio from the 50% expectation than did the paternal transmissions (maternal transmission ration 64.6% vs. paternal 57%).

**Table 8B: rs1861972-rs1861973 parent-of-origin haplotype analysis**

| Population | | | narrow | | | broad | | |
|---|---|---|---|---|---|---|---|---|
| | | | T^{b} | UT^{b} | *P*-value^{c} | T^{b} | UT^{b} | *P*-value^{c} |
| AGRE | Parental | A-C | 204 | 132 | 0.00068 | 215 | 140 | 0.00054 |
| TOTAL^{a} | Transmissions | A-T | 4 | 19 | 0.0011 | 8 | 18 | 0.05489 |
| & NIMH | | G-C | 5 | 11 | 0.1289 | 6 | 11 | 0.2218 |
| | | G-T | 135 | 186 | 0.013 | 140 | 200 | 0.0047 |
| | | global | | | 0.000072 | | | 0.0019 |
| | | | | | | | | |
| | Maternal | A-C | 106 | 58 | 0.00016 | 109 | 65 | 0.00079 |
| | Transmissions | A-T | 2 | 11 | 0.0088 | 4 | 11 | 0.06532 |
| | | G-C | 3 | 5 | 0.4772 | 4 | 5 | 0.7386 |
| | | G-T | 59 | 96 | 0.0028 | 64 | 100 | 0.0047 |
| | | global | | | 0.00025 | | | 0.00446 |
| | | | | | | | | |
| | Paternal | A-C | 98 | 74 | 0.0668 | 106 | 75 | 0.021 |
| | Transmissions | A-T | 2 | 8 | 0.049 | 4 | 7 | 0.3627 |
| | | G-C | 2 | 6 | 0.148 | 2 | 6 | 0.148 |
| | | G-T | 76 | 90 | 0.2769 | 76 | 100 | 0.07 |
| | | global | | | 0.0495 | | | 0.075 |

| | | | | | | | | |
|---|---|---|---|---|---|---|---|---|
| ^{a-} The 167 initial AGRE families and the recently obtained 239 AGRE families are referred to as AGRE TOTAL. ^{b}- T=number of times frequent allele transmitted; U=number of times frequent allele not transmitted from parents. ^{c}- *P*-value generated by TDTPHASE (1df for single haplotype and 3df for the global tests). | | | | | | | | |

A similar pattern of transmission ratios was observed under the broad diagnosis with 215 of 355 A-C heterozygous parents (60.6%) transmitting the A-C haplotype. 106 of 181 (58.7%) were paternal transmissions and 109 of 174 (62.6%) were maternal transmissions, all of which yielded significant global P-values (combined parental *P*=0.00063; paternal *P*=0.034; maternal *P*=0.0045)(Table 8B). These data indicate that ASD-affected individuals are more likely to inherit the maternal A-C *rs1861972-rs1861973* haplotype than the paternal haplotype, suggesting a parent-of-origin effect. Because parent-of-origin effects are indicative of genomic imprinting and because genomic imprinting is regulated by an epigenetic mechanism such as methylation (Delaval and Feil (2004) Curr Opin Genet Dev 14: 188-195; Kaneda et al (2004) Nature 429: 900-903), these results suggest that *EN2* might be genomically imprinted and the epigenetic dysregulation of *EN2* may contribute to ASD.

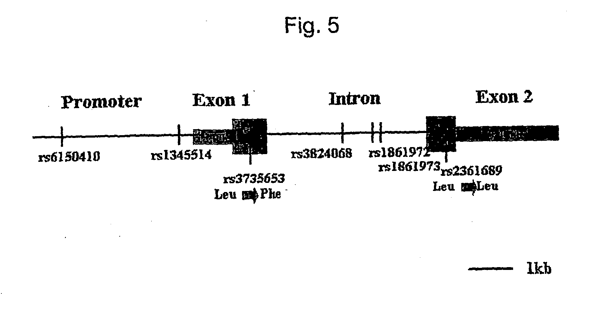

To determine whether other *EN2* polymorphisms also display association with ASD, five other polymorphisms (*rs6150410, rs1345514, rs3735653, rs3824068* and *rs2361689*) that span the majority of the *EN2* gene have been tested for association in the original set of 167 AGRE nuclear pedigrees. The *rs3824068* SNP is located in the intron, 495bp upstream of *ss1861972*. The *rs3735653* SNP is located in exon 1, 2560 bp 5' of *rs1861972*, and alters a Phe for a Leu. The *rs2361689* SNP is located in exon 2, 1187 bp 3' of *rs1861973* and changes the codon usage for Leu. The *rs6150410* and *rs1345514* polymorphisms are located in the promoter, 1799bp and 265bp 5' of the transcriptional start site respectively (Figure 5, which shows a schematic representation of the human *ENGRAILED 2* gene with the location and name of the polymorphisms tested for association with ASD). The frequency of the rare allele for each of these polymorphisms is shown in Table 9.

**Table 9. EN2 SNP characteristics.**

| dbSNP No. | Position | Polymorphism ^{b} | Frequency ^{c} |
|---|---|---|---|
| *rs6150410* | Promoter | CGCATCCCC/- | 0.347 |
| *rs1345514* | Promoter | C/T | 0.345 |
| *rs3735653^{a}* | Exon1 | C/T | 0.489 |
| *rs3824068* | Intron | C/T | 0.375 |
| *rs1861972^{a}* | Intron | C/T | 0.281 |
| *rs1861973^{a}* | Intron | C/T | 0.287 |
| *rs2361689^{a}* | Exon2 | T/C | 0.320 |

| | | | |
|---|---|---|---|
| ^{a}- Frequency of alleles reported previously (Gharani et al (2004) Mol Psychiatry 9:474-484) ^{b}- Second allele is rare allele. ^{c}- Frequency of rare allele. | | | |

Once all genotypes were verified to be in Hardy-Weinberg equilibrium, allelic transmissions were assessed for each polymorphism by PDT version 4.0 or by PDTPHASE version 2.404 program (Dudbridge (2003) Genetic Epidim. 25:115-121), Under the narrow diagnosis, a trend towards over-transmission to affected individuals and over-representation in DSPs of the rare A allele for *rs3824068* was observed but this was not statistically significant (P=0.065) and was not observed under the broad diagnosis (P=0.157) (Table 10). No evidence for association was observed for either *rs6150410, rs1345514, rs3735653* or *rs2361689* under both the narrow or the broad diagnosis (*rs6150410*-narrow: *P*=0.887, broad: *P*= 0.902; *rs1345514*-narrow: *P*=0.581. broad: *P*=0.389)(Table 10).

**Table 10. rs6150410, rs1345514, rs1345514, rs3824068 and rs2361689 PDT and PDTPHASE results in the initial 167 AGRE pedigrees.**

| | | | ParentalTransmissions^{b} | | DSP ^{c} | | | |
|---|---|---|---|---|---|---|---|---|
| Population | SNP | Diagnosis | T | U | A | UA | χ^{2d} | *P*-value ^{e} |
| AGRE ^{a} | *rs61S0410* | narrow | 319 | 317 | 191 | 194 | 0.0022 | 0.9623 |
| | | broad | 398 | 393 | 241 | 245 | 0.0017 | 0.9668 |
| | *rs1345514* | narrow | 271 | 279 | 165 | 170 | 0.2692 | 0.6039 |
| | | broad | 333 | 334 | 211 | 217 | 0.0477 | 0.8271 |
| | *rs3735653* | narrow | 118 | 137 | 111 | 97 | 1.23 | 0.2685 |
| | | broad | 150 | 165 | 144 | 97 | 0.89 | 0.3444 |
| | *rs3824068* | narrow | 282 | 313 | 160 | 170 | 3.424 | 0.0642 |
| | | broad | 362 | 386 | 209 | 219 | 2 | 0.1573 |
| | *rs2361689* | narrow | 119 | 114 | 143 | 115 | 0.02 | 0.8877 |
| | | broad | 146 | 153 | 172 | 117 | 0.39 | 0.5323 |

| | | | | | | | | |
|---|---|---|---|---|---|---|---|---|
| ^{a}- The initial 167 AGRE families. ^{b}- T=number of times frequent allele transmitted; U=number of times frequent allele not transmitted from heterozygous parents. ^{c}- Discordant sibpair counts: A=total number of the common allele in affected siblings; UA=total number of the common allele in unaffected siblings of DSPs. ^{d}- Global χ² values calculated by PDT^{sum} for *rs3735653* and *rs2361689* and by PDTPHASE^{sum} for *rs6150410, rs1345514* and *rs3824068.* ^{e}- *P*-value generated by PDT (1df) for *rs3735653* and *rs2361689* and by PDTPHASE (1df) for *rs6150410*, *rs1345514* and *rs3824068.* | | | | | | | | |

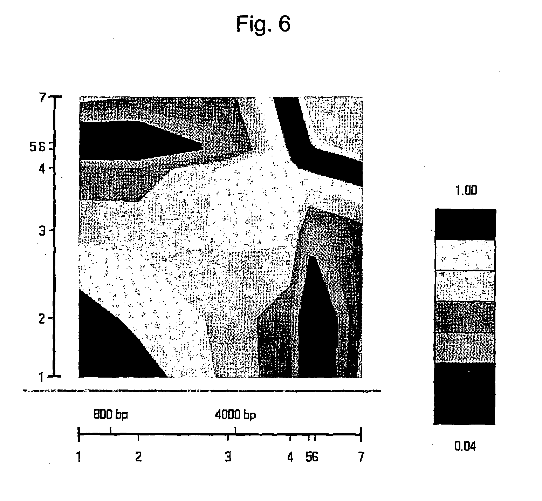

Inter-marker LD relationships were then determined for the 7 polymorphisms that have been studied (Figure 6 and Table 11). As mentioned previously, *rs1861972* and *rs1861973* are in tight LD with each other (D'=0.902). This analysis demonstrates that *rs3824068* is also in strong LD with *rs1861972* (D'=0.899) and *rs1861973* (D'=0.909). The promoter polymorphisms, *rs6150410* and *rs1345514*, are not in LD with *rs1861972* (D'=0.092) and *rs1861973* (D'=0.041) but instead are in strong LD with each other (D'=0.949) (Figure 6 and Table 11). Finally, the exonic SNPs, *rs3735653* and *rs2361689,* display intermediate D' values to both the promoter polymorphisms as well as *rs1861972* and *rs1861973*. These data indicate that two separate LD blocks, one in the promoter, and another in the intron, exist for *EN2* (Figure 6 and Table 11). Figure 6 shows a schematic illustration of the inter-marker linkage disequilibrium values for the seven polymorphisms tested for association in *EN2* gene. Each polymorphism is represented by the following numbers: 1= *rs6150410; 2= rs1345514; 3= rs3735653; 4= rs3824068;* 5= *rs1861972; 6= rs1861973;* 7=*rs2361689*. The position of the polymorphisms within *EN2* is drawn to scale. The color code for LD strength (D') is shown on the right, ranging from 0.04 for weak LD to 1 for strong LD.

**Table 11. Inter-marker LD values for EN2 polymorphisms**

| M1^{a} | M2^{a} | χ2^{b} | Pvalue^{c} | D'^{d} |
|---|---|---|---|---|
| 1 | 2 | 449.20 | <0.00001 | 0.949 |
| 1 | 3 | 100.39 | <0.00001 | 0.694 |
| 1 | 4 | 63.54 | <0.00001 | 0.486 |
| 1 | 5 | 0.52 | 0.47212 | 0.092 |
| 1 | 6 | 0.12 | 0.72877 | 0.041 |
| 1 | 7 | 33.65 | <0.00001 | 0.657 |
| 2 | 3 | 94.59 | <0.00001 | 0.694 |
| 2 | 4 | 54.14 | <0.00001 | 0.472 |
| 2 | 5 | 0.75 | 0.38569 | 0.123 |
| 2 | 6 | 0.37 | 0.54090 | 0.078 |
| 2 | 7 | 22.57 | <0.00001 | 0.585 |
| 3 | 4 | 156.67 | <0.00001 | 0.781 |
| 3 | 5 | 18.21 | 0.00002 | 0.394 |
| 3 | 6 | 23.05 | <0.00001 | 0.421 |
| 3 | 7 | 45.49 | <0.00001 | 0.536 |
| 4 | 5 | 72.07 | <0.00001 | 0.899 |
| 4 | 6 | 84.12 | <0.00001 | 0.909 |
| 4 | 7 | 133.02 | <0.00001 | 1.000 |
| 5 | 6 | 364.64 | <0.00001 | 0.902 |
| 5 | 7 | 24.75 | <0.00001 | 0.634 |
| 6 | 7 | 26.92 | <0.00001 | 0.605 |

| | | | | |
|---|---|---|---|---|
| ^{a}-The name of the polymorphism is represented by the following numerals: 1-*rs6150410*; 2-*rs1345514*; 3*-rs3735653;* 4*-rs3824068*; 5-*rs1861972*; 6-*rs1861973*; *7-rs2361689* ^{b}- χ2 values calculated by GOLD (1999-2001) program ^{c}- *P* values calculated by GOLD (1999-2001) program ^{d}- D' LD values calculated by GOLD (1999-2001) program | | | | |

Finally, 3, 4 and 5 multi-SNP haplotype analysis was performed to investigate whether the A-C *rs1861972-rs1861973* haplotype was consistently associated with ASD. This analysis failed to provide significant evidence for association of any specific haplotype with ASD under both diagnostic criteria. Because *rs3824068* is in strong LD with *rs1861972* and *rs1861973* and shows a trend towards association under the narrow diagnosis, this SNP was now included in multi-SNP haplotype analysis. In a three SNP haplotype analysis with *rs1861972* and *rs1861973,* seven of the eight different haplotypes are observed in the population but none of them are significantly associated with ASD under both diagnostic criteria. Two haplotypes contain the associated core A-C *rs1861972-rs1861973* haplotype. The T-A-C haplotype is minimally associated with ASD under the narrow diagnostic criteria but not under the broad diagnosis (narrow: *P*=0.037; broad: *P*=0.123) while the C-A-C is not associated with ASD under both diagnostic criteria (narrow: *P*=0.736; broad: *P*=0.404)(Table 12).

**Table 12. rs3824068-rs1861972-rs1861973 haplotype analysis: PDTUNPHASE results under the narrow and the broad diagnosis.**

| | | | Parental Transmissions ^{b} | | DSP ^{c} | | | |
|---|---|---|---|---|---|---|---|---|
| Population | Haplotype | Diagnosis | T | U | A | UA | χ^{2d} | *P*-value ^{e} |
| AGRE^{a} | C-T-C | narrow | 160 | 158 | 91 | 86 | | 0.7356 |
| | C-T-T | | 5 | 17 | 3 | 0 | | - |
| | C-C-C | | 4 | 9 | 4 | 4 | | - |
| | C-C-T | | 105 | 124 | 51 | 69 | | 0.5258 |
| | T-T-C | | 192 | 158 | 106 | 95 | | 0.0366 |
| | T-T-T | | 3 | 5 | 2 | 2 | | - |
| | T-C-T | | 3 | 1 | 1 | 0 | | - |
| | Global | | | | | | 7.514 | 0.0572 |
| | | | | | | | | |
| | C-T-C | broad | 205 | 196 | 116 | 105 | | 0.4043 |
| | C-T-T | | 8 | 20 | 3 | 0 | | - |
| | C-C-C | | 5 | 10 | 4 | 4 | | - |
| | C-C-T | | 132 | 149 | 69 | 91 | | 0.0816 |
| | T-T-C | | 224 | 197 | 123 | 114 | | 0.123 |
| | T-T-T | | 3 | 5 | 2 | 2 | | - |
| | T-C-T | | 3 | 3 | 1 | 1 | | - |
| | Global | | | | | | 6.289 | 0.0983 |

| | | | | | | | | |
|---|---|---|---|---|---|---|---|---|
| ^{a}- The initial 167 AGRE families. ^{b}- T=number of times frequent allele transmitted; U=number of times frequent allele not transmitted from heterozygous parents. ^{c}- Discordant sibpair counts: A=total number of the common allele in affected siblings; UA=total number of the common allele in unaffected siblings of DSPs. ^{d}- Global χ² values calculated by PDTPHASE^{sum} ^{e}- *P*-value generated by PDTPHASE^{sum} (1df for single haplotype and 3df for the global tests). | | | | | | | | |

These data provide evidence that the A-C *rs1861972-rs1861973* haplotype is not always associated with ASD. Four SNP haplotype analysis for *rs3735653-rs1861972-rs1861973-rs2361689* confirms that the A-C *rs1861972-rs1861973* haplotype is not always associated with ASD (Table 13). Together all our data indicate that the A and C alleles of *rs1861972* and *rs1861973* are unlikely to be functional and that the putative etiological variant is in strong LD with both *rs1861972* and *rs1861973.* Further LD-mapping and association analysis of other variants in the region will map and identify the etiological variant.

**Table 13. rs3735653, rs1867972, rs1867973 and rs2361689 haplotype PDT results in 146 extended pedigrees analyzed under the narrow and broad diagnoses.**

| | | Parental | | | | | |
|---|---|---|---|---|---|---|---|
| | | Transmissions^{b} | | | DSP^{c} | | |
| Haplotype^{a} | Diagnosis | T | U | A | UA | χ2^{d} | *P*-value^{e} |
| C-A-C-T | narrow | 45 | 34 | 23 | 21 | - | 0.3800 |
| T-A-C-T | | 119 | 86 | 65 | 45 | - | 0.0402 |
| T-G-C-T | | 3 | 6 | 2 | 2 | - | 0.1573 |
| C-A-T-T | | 1 | 5 | 1 | 0 | - | 0.0896 |
| T-A-T-T | | 1 | 5 | 2 | 1 | - | 0.2059 |
| C-G-T-T | | 53 | 63 | 28 | 27 | - | 0.2004 |
| T-G-T-T | | 19 | 29 | 9 | 10 | - | 0.2050 |
| C-A-C-C | | 72 | 78 | 47 | 36 | - | 0.8658 |
| T-A-C-C | | 32 | 23 | 13 | 10 | - | 0.3402 |
| C-G-C-C | | 1 | 4 | 1 | 0 | - | 0.4142 |
| C-A-T-C | | 1 | 6 | 1 | 1 | - | 0.2253 |
| C-G-T-C | | 11 | 12 | 3 | 6 | - | 0.5791 |
| T-G-T-C | | 1 | 1 | 1 | 1 | - | 0.3173 |
| Global | | | | | | 17.63 | 0.1276 |
| | | | | | | | |
| C-A-C-T | broad | 57 | 42 | 33 | 21 | - | 0.2924 |
| T-A-C-T | | 137 | 112 | 70 | 46 | - | 0.1532 |
| T-G-C-T | | 3 | 9 | 2 | 2 | - | 0.1573 |
| C-A-T-T | | 3 | 10 | 1 | 0 | - | 0.1573 |
| T-A-T-T | | 2 | 5 | 2 | 1 | - | 0.4054 |
| C-G-T-T | | 67 | 77 | 34 | 27 | - | 0.2322 |
| T-G-T-T | | 25 | 37 | 12 | 11 | - | 0.1228 |
| C-A-C-C | | 88 | 95 | 56 | 36 | - | 0.8383 |
| T-A-C-C | | 43 | 29 | 21 | 10 | - | 0.1093 |
| C-G-C-C | | 1 | 5 | 1 | 0 | - | 0.3173 |
| C-A-T-C | | 1 | 7 | 1 | 1 | - | 0.1797 |
| C-G-T-C | | 15 | 14 | 6 | 6 | - | 0.7456 |
| T-G-T-C | | 1 | 1 | 1 | 1 | - | 0.3173 |
| Global | | | | | | 16.76 | 0.1587 |

| | | | | | | | |
|---|---|---|---|---|---|---|---|
| ^{a}- Haplotypes recoded as alleles prior to PDT analysis. ^{b}- Parental Transmissions: T=number of times common allele transmitted and U=number of times common allele not transmitted from heterozygous parents. A total of 271 triads were included in the analysis. ^{c}- Discordant sibpair counts: A= total number of the common allele in affected siblings and UA-total number of the common allele in unaffected siblings of genotypic discordant DSPs. A total of 151 DSPs were included in the analysis. ^{d}- Global χ2 values calcuated by PDT^{sum} (26). ^{e}- *P*-value generated by PDT (1df for single haplotype *P*-values and 12df for the global test). | | | | | | | |

## Claims

1. An *in vitro* diagnostic method for determining the predisposition, the onset or the presence of autism spectrum disorder in a person, said method comprising detecting in said person the existence of a change in an intronic segment of the genome, which segment is located at least within the portion of chromosome 7 as set forth in SEQ ID NO:1, and wherein said change comprises a G to A transition at position 5579 and/or a T to C transition at position 5731 of SEQ ID NO:1.

## Patentansprüche

1. Eine diagnostische *In-vitro*-Methode zur Bestimmung der Prädisposition, des Ausbruchs oder des Vorliegens von Autismus-Spektrum-Störungen bei einer Person, wobei die besagte Methode bei der besagten Person den Nachweis einer bestehenden Veränderung eines intronischen Segments des Genoms beinhaltet und sich dieses Segment wenigstens innerhalb des Abschnitts des Chromosoms 7, wie in SEQ ID Nr. 1 dargelegt, befindet, und worin die besagte Veränderung eine Transition von G zu A an der Position 5579 und/oder eine Transition von T zu C an der Position 5731 von SEQ ID Nr. 1 beinhaltet.

## Revendications

1. Une méthode de diagnostic *in vitro* pour la détermination de la prédisposition, de l'apparition ou de la présence de troubles du spectre autistique chez un sujet, ladite méthode consistant en la détection chez ladite personne de l'existence d'un changement dans un segment intronique du génome, le segment étant situé au moins dans la partie du chromosome 7 selon la séquence SEQ ID NO:1, et dans laquelle ledit changement consiste en une transition G à A à la position 5579 et/ou en une transition T à C à la position 5731 de la séquence SEQ ID NO:1.


### SEQUENCE LISTING

<110> University of Medicine and Dentistry of New Jersey Rutgers, the State University of New Jersey
   Millonig, James H. Brzustowicz, Linda
   Gharani, Neda
<120> Genes as diagnostic tools for autism
<130> CABM-03-16PCT
<140> New PCT Application
   <141> 2004-06-02
<150> 60/484,633
   <151> 2003-07-03
<160> 12
<170> PatentIn version 3.2
<210> 1
   <211> 10088
   <212> DNA
   <213> Homo sapiens
<400> 1
   ctctcaccag ccctattttc ccggggggca ggtagactgt ggggtttgat tttttgagac     60
   tcggggagga cccctggcag atgtgtgcct agccagaaca tttggtaagg actcctccaa    120
   tgaagaaaaa gtggaggaat ccagccccag cgagaagagg cttccccacc ctgccctaga    180
   cacaccggac agagggcaca ctctgaccag agccacgtcc agtggccagg aggccagccc    240
   agcacctcct cccccaccat tcccgtcctg ggtggggggg taattttcct gggagcagct     300
   gtgggaactg tcgtttctca tcccagccca gccagcactc tgaagcctgc aggggaagga    360
   cagcacgtgg gatggacact ggggaaggag cttcgcaagg ccagggtgca accttcaggc    420
   cccaggtggc ctggcaggcc acgctgcctc ggagatgctt gccagactcc ccaagttcac    480
   tcagggcctg gcagcaacct gctggctgcc tctgcggggg tctgggttgc tgagcacggt    540
   gcagctgccc agggccaatc agccctaggg tgtccgtgcc aggctgcggc ctccccgcct    600
   cccccgcact gagggtactc atggcgtgca aatgctcccg cacccccaga gctgccctat    660
   cggatgtttc caggaattca cacatttcca taaaaatgca ttttaaatga tggacaggcg    720
   agcctggggt aacaacgggt gtttggtggg tagacaagag caaatgggaa ggagcccgag    780
   ggaggagggg gaagagaaga ggaaacagaa cttccagttg gacattctga caacagctgg    840
   aaggaaagtc tagaaaagat gaagagagag gaggggagaa accaactggg gctcccaccc    900
   ttgccgttgg attcctaatt ctcgtttcaa atgggccctg ctctccggca aaattagttt    960
   aaaggatttt aaaacaaaga aaacgagatg accggtctgg gagctcctca atcagagtag   1020
   agaagttaga ggggggcggg cgacttggtt ttgaagtctt agctgaacag tcacccctcc    1080
   tctccttggc aaaaaggatt cctttagaac ctccgaggct cctggatttc tcccttcgca   1140
   aatggagccg catactgcat tcccccgctc tttcggatcg ctaagcatgt ttcatgaggg   1200
   tcgctgtccc cgggtggaat gcggccgtat gcacgcgcct ccctgcacac gcacacacac 1260
   gcacacttac aataagtgtc tgcaggagga gtgtcctgcg cgccagctct gcgtttaaga 1320
   caggaagctg ccgggttacc gagtcaaatg ggagtgacac tattcctctc catcagcaag 1380
   gaaagcggac cacaaaagtc cctttgtatc tcggcagctc atttaatatt atttatgcat 1440
   tttgtgcaag gaattgtggg atttcgcccc acggtaaaca atatggaaat cttaaaaata 1500
   gcgatcttcc tgtgcgtgtc cacctacgcg ccccggggtg acctggcggg gctgtcgccg 1560
   ggtgactcac acccctgaac cgcgaagcga cagggaaagc gcgggcgagc gcaggagacg 1620
   cggtcggggg tctctccggg ttcctgggct cccgcacccg gagcggggga cgcggccgct 1680
   ttaaggggag gaggggcggc gggctgctcc tgtcacccag cggcggccgg agcgtcacgt 1740
   gggcgcgcgg cgccgcggcc attggcccga ggcacgtgtc caggagaccg gcctgcgacg 1800
   tcactcgagg gggctctgtt aaaaataaga acaaaaatcc agagtgaaag tgtctcaggt 1860
   tgcgccgagt ggcctggaaa tttccgagcc cgcgcggagg ccgaggcggc gagggcggcg 1920
   gacggccggg gagcgcgggc ggcccagccc ggcccggccg ggccctggcc tcgcgtctct 1980
   cacccatgcg actcgggccg cggagctctg cggggctcgg cgggggcgcg gccgcacgcc 2040
   ggtggggcgc cccggcccgc agcggggcgg cggccgcgag gagggggcct ccatgtgcgt 2100
   gcgggcggtg gcgggcgcgc tgaccgcggg cgcccggcac cctcgagggc cggctagggc 2160
   gtgcgggcgg ggacggccgg gcggcggcgg cggccggagc cggcccgggc gggcgtgagc 2220
   gccggggaac gcgctgcctg catgcgcgca gctctcgccc cgggcggccc aggcggcggc 2280
   gccggagccc gaggcggccg gacgcggaga ggagcgggga gcccgggagg cggcccgcgt 2340
   ccccgccgga ccactgcgac tgtctagacc ccggctgcgc ggcgaagtcg aggacttggc 2400
   tctgttgaat ctctcatcgt ctgggcgagc ggggcggctc gtggtgtttc taacccagtt 2460
   cgtggattca aaggtggctc cgcgccgagc gcggccggcg acttgtagga cctcagccct 2520
   ggccgcggcc gccgcgcacg ccctcggaag actcggcggg gtgggggcgc gggggtctcc 2580
   gtgtgcgccg cgggagggcc gaaggctgat ttggaagggc gtccccggag aaccagtgtg 2640
   ggatttactg tgaacagcat ggaggagaat gaccccaagc ctggcgaagc agcggcggcg 2700
   gtggagggac agcggcagcc ggaatccagc cccggcggcg gctcgggcgg cggcggcggt 2760
   agcagcccgg gcgaagcgga caccgggcgc cggcgggctc tgatgctgcc cgcggtcctg 2820
   caggcgcccg gcaaccacca gcacccgcac cgcatcacca acttcttcat cgacaacatc 2880
   ctgcggcccg agttcggccg gcgaaaggac gcggggacct gctgtgcggg cgcgggagga 2940
   ggaaggggcg gcggagccgg cggcgaaggc ggcgcgagcg gtgcggaggg aggcggcggc 3000
   gcgggcggct cggagcagct cttgggctcg ggctcccgag agccccggca gaacccgcca 3060
   tgtgcgcccg gcgcgggcgg gccgctccca gccgccggca gcgactctcc gggtgacggg 3120
   gaaggcggct ccaagacgct ctcgctgcac ggtggcgcca agaaaggcgg cgaccccggc 3180
   ggccccctgg acgggtcgct caaggcccgc ggcttgggcg gcggcgacct gtcggtgagc 3240
   tcggactcgg acagctcgca agccggcgcc aacctgggcg cgcagcccat gctctggccg 3300
   gcgtgggtct actgtacgcg ctactcggac cggccttctt caggtgagcc cgcggggacc 3360
   acgcgtcccg gctcgccgcg gggaggcccg cggagctggg gggcggtgct ggcgcgggaa 3420
   cttaccggga ggaaaacatc tcgaacctcc cccgcgcaca cgcacaaaga ctcacgcgac 3480
   attgtgtgaa gctgacgccg gcccgggcag cggccaggag tccagcggca ggactgattc 3540
   gctagggggt acagacttct taggaccgca gaagggacct tctttctttc tctgtctctc 3600
   tctctccttc ctctctccct gtctcccctc tctgtcttcc accccgcctt ggcgcatctc 3660
   ttcccagccc ctagcccatg tctcccccac tgtagttttt tttggtggga acgtggtggc 3720
   tggaagatgg gcccggaagt gcacactctc atccccttcc ctacgatctt ccaactcagg 3780
   ccaggccggg gacgcatgcc ccagcccacc ccagacttgt cctaccatcc ggttatcccg 3840
   gctgtgctcg gggaagaaaa ggcgaggccc tttgccgctc tgccttctgc ccctcgggcc 3900
   tgcgctgacc ggtgggactc aggaggatgc acacagggaa ggaggaaaat aaaggcgccc 3960
   tttccccttg gctccacttt gtttgccagc gccagcccgc agtggtgggg ctcagccccc 4020
   ttcctgcaca cagcgaggac aagggaggca gccgtccctc ccggcacctg ccatccccaa 4080
   atagaaagga ccttctctca gggtttcctg ggggctgctg atgggaaaga ggcagcattc 4140
   gcaggggccc tgcagagatg ctggatatat tttttcatag atctgcgatt ttaaaaaact 4200
   aagtccatgt ccttgtagaa atcatcaact gcattccatg cgggtctgcg gctgggaacc 4260
   gccattagaa gtggactgtt tgaccccgag ctggcagcgg atccccgctg cccccaaacc 4320
   ctcaactatt ttgcgggggt catttgccca gatcacagca ggagtgagcc aacccttggg 4380
   ccgccatccc gcagaactat gcgtgcatat ttctgatgaa attcagattt ctcagctaga 4440
   tctgaaattt gctccattgt tctcgtcttc ctcctttgtt aatatttaat taacatacag 4500
   gctcacaatg ccgggcgagg agactcggcc gggctttgtg cggcgcggga gttcgctgag 4560
   ccagccccca acggcccggg agctgggcag caccgcccgg cccggcctgg cccggcccag 4620
   ctcagcccag cccaagtcgc ctatcttcat gggctttaaa atatccctgc aagataatgt 4680
   ttctgttctt tggtttctcc gaaagaaagg ggagagagag ttttcttggg gaggtttgat 4740
   tctgtttctg agactcctaa gtatttgtct tttgaaagaa aatcaagaaa aaaacctaaa 4800
   aattattatt ttagggaaat ttattgccat aaaatggtgt ccttttgcgg gctgcttcat 4860
   gagtgcatta acaagagccc caggaccaga agagtttggg ggtagagttc tggggaaggg 4920
   agtggttgga aacccagaca gagatgggct ctgggagcag gaggctgggg cctctcctgg 4980
   agccttgtgc cccatccccc accaccgttc cggagggcca accccatctc caaatctgca 5040
   cctaccccta ccaaagccag gcccactggc ctggagccct gggcgtgagc aagacaggca 5100
   ctgagtgtgt acgtgtgcat ggggtgggtg cccaagtata gggtgtgtgc tctcatgggt 5160
   ggtgagcctg cctatgggtt gctttaaaag ctgcccttgg tgctcctgag gtggtgccca 5220
   cagatcctct ttctccaggc ctgtctcctg gagagagcac aagactcact tggccatgag 5280
   ggagtgcttg gcattcaccc tgggcctcca gttcgtcccc cacctcttgc tgggcacagc 5340
   cccagcaccc tagttgactc tcctgacctg ggcagggtgc agtcccaggg cctccaagga 5400
   gatccacatt cctcttctcc tcagtgtgcc cggcagctct ccggccctga agggtggggg 5460
   gcccccagcc ttctccagcc acagggacct gtgatgaagc tggggccaga tgctccctaa 5520
   agccgattca tacaccgcac aaattgaaac ccagaggcga ggtcaccact ccctgccagt 5580
   ggccttgccc ccttcttccc ccacagggaa cgccaggggg ttgagcctct tatcaccaaa 5640
   aagaaactga tgacacttcc ctccttctgc tctcctccct ctgccctttc cccatggata 5700
   gcaggtccta gaagccttac agcgaccctg tccaaaacct ggggcaggtc cacagggaga 5760
   aggccaggtc aggttcataa gtctgaatcc cagttgggag gcacagtggg gagggtcaga 5820
   agtggacctg gacaaggtca gctgggctac cctgctgccc acagtgaagc agtcccatgt 5880
   ctggggaaag ggtggtgcag tcaacaccct tgtagagcca ggtcccctct cctggacagg 5940
   aaacctggga gatttccagt gggtgaagga ctcacccact gtgagtagct cagtgcccct 6000
   ccccaccaag gagggaagta catgcactga ctcttcctta aaggaatgaa cttgggtcca 6060
   cagagccctt ggctgggagt catagaggag cttgggtgga ggcagacact ctgggcccct 6120
   cctgtcctca gggcccacct gcccctgatt cccacagccc ttggcaccct gtggggtatc 6180
   cccatgaggg tccccatcat agccctcagg gcgcctcctg cttctgtgac cgccctgcag 6240
   cctttcccag tcttctctcc tcccctctct ctcagcacct accgccatcc ctgttcctga 6300
   acagagagcc tcagaaagga ctaggaaaaa ccaggccaga aagtgtgggg agttttgctt 6360
   acatccagga gttttacttt tatctagaga cccctcattt gtggtcagcc tgcccactag 6420
   gcctgcccca tagtcccact tacatcacac gcagtcctcc ctcaccaagt ggtggaggtc 6480
   cgcgttgagc ccatgcccag tccgaagtct agccccacat ctggcggttc agcctcgagt 6540
   ggccttgggc gagtcacttc cctcttgggg ccccagaatc cttacccggt gcctattggg 6600
   tggcacacct ctgggtaacc tgacttctct ctgtccaccg tatctaccca ggtcccaggt 6660
   ctcgaaaacc aaagaagaag aacccgaaca aagaggacaa gcggccgcgc acggccttta 6720
   ccgccgagca gctgcagagg ctcaaggccg agttccagac caacaggtac ctgacggagc 6780
   agcggcgcca gagcctggcg caggagctga gcctcaacga gtcacagatc aagatttggt 6840
   tccagaacaa gcgcgccaag atcaagaagg ccacgggcaa caagaacacg ctggccgtgc 6900
   acctcatggc acagggcttg tacaaccact ccaccacagc caaggagggc aagtcggaca 6960
   gcgagtaggg cggggggcat ggaggccagg tctcagtccg cgctaaacaa tgcaataatt 7020
   taaaatcata aagggccagt gtataaagat tataccagca ttaatagtga aaatattgtg 7080
   tattagctaa ggttctgaaa tattctatgt atatatcatt tacaggtggt ataaaatcca 7140
   aaatatctga ctataaaata tttttttgag ttttttgtgt ttatgagatt atgctaattt 7200
   tatgggtttt tttctttttt gcgaaggggg ctgcttaggg tttcaccttt ttttaatccc 7260
   ctaagctcca ttatatgaca ttggacactt ttttattatt ccaaaagaag aaaaaattaa 7320
   aacaacttgc tgaagtccaa agatttttta ttgctgcatt tcacacaact gtgaaccgaa 7380
   taaatagctc ctatttggtc tatgacttct gccactttgt ttgtgttggc ttggtgagga 7440
   cagcaggagg ggcccacacc tcaagcctgg accagccacc tcaaggcctt ggggagctta 7500
   ggggacctgg tgggagagag gggacttcca gggtccttgg gccagttctg ggatttggcc 7560
   ctgggaagca gcccagcgta ccccaggcct gctctgggaa gtcggctcca tgctcaccag 7620
   cagccgccca ggcccgcagc ctcacccggc tccctctcct caccctcctg cacctaactc 7680
   cctcctcctt ctcctttttc ctcctcttcc tccttcctcc ttcctcctgc tcctcctttc 7740
   ttcttctttt tcttctcctc ctcctccttc cttcctcctc ctccttctct ttcctcctcc 7800
   tcctcaccaa gggcccaacc gtgtgcatac atcgtctgcg tctgtggtct gtgtcgctgt 7860
   ccccagtccc accgcagtcc tgccgcaggc ctaaccctcc tgccctgggc actgcctcca 7920
   tgcagaagcg cttcgaggtt ctggggctaa aggcctgggg tgtgtggcct aaagcccaag 7980
   agcggtgggg cgaccctcct tttggcttgg ccccaggaat ttcctgtgac tccaccagcc 8040
   atcatgggtg ccagccaggg tcccagaaat gaggccatgg ctcactgttt ctgggcgggc 8100
   agaaggctct gtagagggag atggcatcat ctatcttcct ttcctttttc ttttcttccc 8160
   tatttttttc tttttttcct ttattttttt cttttcttgg agtggctgct tctgctatag 8220
   agaacattct tccaagataa atatgtgtgt ttacacatat gtctgcatgc atgtgaacac 8280
   acacacacac acacacacac caggcgtgtt tgagtccaca gttctgaaac atgtggctac 8340
   cttgtctttc aaaagaactc agaatcctcc aggatctaga agaaggaaga aagtgtgtaa 8400
   ataatcattt cttatcatca ctttttgtct tttcttgttt tttaaaatat acattttatt 8460
   tttgaaggtg tggtacagtg taaattaaat atattcaata tatttcccac caagtaccta 8520
   tatatgtata taaacaaaca cattatctat atataacgcc acactgtctt ctgtttagtg 8580
   tatggggaaa gaccaatcca actgtccatc tgtggctggg acagcccagg gggtgtgccc 8640
   acggctgacc caggggtgtg cacacggctg agctgggagt cccgctggtc tccctgagga 8700
   ctgagggtga acttcgctct ttgccttaaa cctctttatt tcattgcagt aatagtttta 8760
   cgttgtacat aatagtgtaa acctttttaa aaaggaaagt ataaaaacaa aagttgtaat 8820
   ttaaaagtct gaataaccat ctgctgctta ggaaactcaa tgaaatgaca tgccttttta 8880
   gcaggaagca aagttggttt ctgttttttg ttttctttgt tgttttagtt tataaaacat 8940
   gtgcatttta cagttccagt atcaaatatt tataatctta tgagaaatga atgaatgttt 9000
   ctatttacaa ctgtgcttat caaaattgtg aacaccccca cccccgcatt tttgtgtgtt 9060
   gaaattcttg aaggttacat taaataaaac aaaatctctt tattataaaa tactgaaggt 9120
   cccaggtgga atgatgagtt ttctaagcat gtctgagtcc agaggaactg cctgggcatc 9180
   ctccccagta ctcaggtgct catggccttc gccccgcctg ggagcaggtg ccaggtgcct 9240
   ttgtagcgag aggtgattat tatttttttt taaataagac agtttggata tgtcacatgt 9300
   gagcacagaa taaccaaaag aagtacacat tttgttacca ggaggaagtg ttcaaaacta 9360
   cggtgctatt ccagtgacta cattttttta aattttgcga agttcaacag gtctccctcc    9420
   ggctgggtgc tcactggccc tgccacccgt gctggaccga ggcgctttcc cctggggaca    9480
   ggaacttttt actccactga gacaaagaga ctctcctttt caaagtgtca tttttgttta    9540
   ccttttgttg tttccccccc aaaacaaaag actttgtaca acttcctaag caggattggg   9600
   ggagggatca gcttcgcctg tgctgaccgc gcctctggat tccgctcatc aggaagtgtt    9660
   tggggtcttt gagcgagtga tctacgcgtt gaattgctgt ttttccaaac tttcatggct    9720
   taaaaatttt ttttcttctc cctgtaaaag aaactcagag actggtgatc tgttaccgaa    9780
   gacgactttt taggcgtttt ctgtttcccc aggcagctgg gccgttggcg ttcccaggcg    9840
   gaagcggggc ctctgggctg ggcctctgcc ctgccaggaa gcggtctggg ggctgcccct    9900
   cgggattttc agaaccctgg aaggagagag ctggggtctc accgcggaga ctcccgagtt    9960
   ctcgttgcct gggcccgagc tgcgccccag ggtcatcgct gatggcgcga gaccacgcat   10020
   tccccgcgaa ccccgacttc tgaacagttc agaaagttcg tagattaaaa accaaacaaa   10080
   aaatactg                                                            10088
<210> 2
   <211> 333
   <212> PRT
   <213> Homo sapiens
<400> 2
   Met Glu Glu Asn Asp Pro Lys Pro Gly Glu Ala Ala Ala Ala Val Glu
   1               5                   10                  15
   Gly Gln Arg Gln Pro Glu Ser Ser Pro Gly Gly Gly Ser Gly Gly Gly
               20                  25                  30
   Gly Gly Ser Ser Pro Gly Glu Ala Asp Thr Gly Arg Arg Arg Ala Leu
           35                  40                  45
   Met Leu Pro Ala Val Leu Gln Ala Pro Gly Asn His Gln His Pro His
       50                  55                  60
   Arg Ile Thr Asn Phe Phe Ile Asp Asn Ile Leu Arg Pro Glu Phe Gly
   65                  70                  75                  80
   Arg Arg Lys Asp Ala Gly Thr Cys Cys Ala Gly Ala Gly Gly Gly Arg
                   85                  90                  95
   Gly Gly Gly Ala Gly Gly Glu Gly Gly Ala Ser Gly Ala Glu Gly Gly
               100                 105                 110
   Gly Gly Ala Gly Gly Ser Glu Gln Leu Leu Gly Ser Gly Ser Arg Glu
           115                 120                 125
   Pro Arg Gln Asn Pro Pro Cys Ala Pro Gly Ala Gly Gly Pro Leu Pro
       130                 135                 140
   Ala Ala Gly Ser Asp Ser Pro Gly Asp Gly Glu Gly Gly Ser Lys Thr
   145 150 155 160
   Leu Ser Leu His Gly Gly Ala Lys Lys Gly Gly Asp Pro Gly Gly Pro
   165 170 175
   Leu Asp Gly Ser Leu Lys Ala Arg Gly Leu Gly Gly Gly Asp Leu Ser
   180 185 190
   Val Ser Ser Asp Ser Asp Ser Ser Gln Ala Gly Ala Asn Leu Gly Ala
   195 200 205
   Gln Pro Met Leu Trp Pro Ala Trp Val Tyr Cys Thr Arg Tyr Ser Asp
   210 215 220
   Arg Pro Ser Ser Gly Pro Arg Ser Arg Lys Pro Lys Lys Lys Asn Pro
   225 230 235 240
   Asn Lys Glu Asp Lys Arg Pro Arg Thr Ala Phe Thr Ala Glu Gln Leu
   245 250 255
   Gln Arg Leu Lys Ala Glu Phe Gln Thr Asn Arg Tyr Leu Thr Glu Gln
   260 265 270
   Arg Arg Gln Ser Leu Ala Gln Glu Leu Ser Leu Asn Glu Ser Gln Ile
   275 280 285
   Lys Ile Trp Phe Gln Asn Lys Arg Ala Lys Ile Lys Lys Ala Thr Gly
   290 295 300
   Asn Lys Asn Thr Leu Ala Val His Leu Met Ala Gln Gly Leu Tyr Asn
   305 310 315 320
   His Ser Thr Thr Ala Lys Glu Gly Lys Ser Asp Ser Glu
   325 330
<210> 3
   <211> 24
   <212> DNA
   <213> Artificial
<220>
   <223> Forward outer primer for intron 1 A/G
<400> 3
   tgaagctggg gccagatgct ccta 24
<210> 4
   <211> 25
   <212> DNA
   <213> Artificial
<220>
   <223> Reverse outer primer for intron 1 A/G
<400> 4
   catggggaaa gggcagaggg aggag 25
<210> 5
   <211> 25
   <212> DNA
   <213> Artificial
<220>
   <223> Forward inner primer for Intron 1/AG
<400> 5
   aggcgaggtc accactccct gcaag 25
<210> 6
   <211> 24
   <212> DNA
   <213> Artificial
<220>
   <223> Reverse inner primer for intron 1 A/G
<400> 6
   gggggaagaa gggggcaagg caat 24
<210> 7
   <211> 22
   <212> DNA
   <213> Artificial
<220>
   <223> Forward outer primer for intron 2 C/T
<400> 7
   gccagggggt tgagcctctt at 22
<210> 8
   <211> 21
   <212> DNA
   <213> Artificial
<220>
   <223> Reverse outer primer for intron 2 C/T
<400> 8
   caggtccact tctgaccctc c 21
<210> 9
   <211> 21
   <212> DNA
   <213> Artificial
<220>
   <223> Forward inner primer for intron 2 C/T
<400> 9
   gaagccttac agcgacccgg t 21
<210> 10
   <211> 20
   <212> DNA
   <213> Artificial
<220>
   <223> Reverse inner primer for intron 2 C/T
<400> 10
   gacctgcccc aggtttttgg 20
<210> 11
   <211> 501
   <212> DNA
   <213> Homo sapiens
<400> 11
   agccccagca ccctagttga ctctcctgac ctgggcaggg tgcagtccca gggcctccaa 60
   ggagatccac attcctcttc tcctcagtgt gcccggcagc tctccggccc tgaagggtgg 120
   ggggccccca gccttctcca gccacaggga cctgtgatga agctggggcc agatgctccc 180
   taaagccgat tcatacaccg cacaaattga aacccagagg cgaggtcacc actccctgcc 240
   artggccttg ccccttcttc cccccacagg gaacgccagg gggttgagcc tcttatcacc 300
   aaaaagaaac tgatgacact tccctccttc tgctctcctc cctctgccct ttccccatgg 360
   atagcaggtc ctagaagcct tacagcgacc ctgtccaaaa cctggggcag gtccacaggg 420
   agaaggccag gtcaggttca taagtctgaa tcccagttgg gaggcacagt ggggagggtc 480
   agaagtggac ctggacaagg t 501
<210> 12
   <211> 497
   <212> DNA
   <213> Homo sapiens
<400> 12
   agccccagca ccctagttga ctctcctgac ctgggcaggg tgcagtccca gggcctccaa    60
   ggagatccac attcctcttc tcctcagtgt gcccggcagc tctccggccc tgaagggtgg   120
   ggggccccca gccttctcca gccacaggga cctgtgatga agctggggcc agatgctccc   180
   taaagccgat tcatacaccg cacaaattga aacccagagg cgaggtcacc actccctgcc   240
   agtggccttg cccccttctt cccccacagg gaacgccagg gggttgagcc tcttatcacc   300
   aaaaagaaac tgatgacact tccctccttc tgctctcctc cctctgccct ttccccatgg   360
   atagcaggtc ctagaagcct tacagcgacc ctgyccaaaa cctggggcag gtccacaggg   420
   agaaggccag gtcaggttca taagtctgaa tcccagttgg gaggcacagt ggggagggtc   480
   agaagtggac ctggaca                                                  497